# EUROPEAN PATENT SPECIFICATION

(11) **EP 3 936 509 B1**
(45) Date of publication and mention of the grant of the patent: **31.07.2024**
(21) Application number: 20771072.4
(22) Date of filing: 06.03.2020
(51) Int. Cl.: C07D 471/14, C07D 519/00, A61P 35/00, A61K 31/438, A61K 31/4995, A61K 31/4375, A61K 31/496, A61K 31/499, A61K 31/5386, A61K 31/5377, A61K 31/541

(54) **FGFR4 KINASE INHIBITOR, PREPARATION METHOD THEREFOR AND USE THEREOF**
FGFR4-KINASEINHIBITOR, HERSTELLUNGSVERFAHREN DAFÜR UND VERWENDUNG DAVON
INHIBITEUR DE FGFR4 KINASE, SON PROCÉDÉ DE PRÉPARATION ET SON UTILISATION

(30) Priority: 08.03.2019 CN 201910178024; 05.07.2019 CN 201910602669
(43) Date of publication of application: 12.01.2022
(73) Proprietor: Shouyao Holdings (Beijing) Co., Ltd., Beijing 100176 (CN)
(72) Inventor: WANG, Jinghan, Beijing 100195 (CN); YAO, Jinsuo, Beijing 100195 (CN); DUAN, Xiaowei, Beijing 100195 (CN); YUAN, Baokun, Beijing 100195 (CN); LIU, Xijie, Beijing 100195 (CN); JIAO, Nan, Beijing 100195 (CN); MIN, Wangyang, Beijing 100195 (CN); SUN, Ying, Beijing 100195 (CN); LU, Chang, Beijing 100195 (CN); SUN, Yinghui, Beijing 100195 (CN); ZHANG, Jiuqing, Beijing 100195 (CN); JI, Yeling, Beijing 100195 (CN); HOU, Deng, Beijing 100195 (CN)
(74) Representative: Hoffmann Eitle
(86) International application number: PCT/CN2020/078098
(87) International publication number: WO 2020/182062

(56) References cited:
- WO-A1-2014/144737
- WO-A1-2017/050864
- WO-A1-2018/004258
- WO-A1-2018/004258
- WO-A1-2018/113584
- WO-A1-2018/153373
- WO-A2-2014/011900
- CN-A- 1 646 529
- HAGEL MARGIT ET AL: "First Selective Small Molecule Inhibitor of FGFR4 for the Treatment of Hepatocellular Carcinomas with an Activated FGFR4 Signaling Pathway", CANCER DISCOVERY, vol. 5, no. 4, 5 April 2015 (2015-04-05), US, pages 424 - 437, XP055857952, ISSN: 2159-8274, Retrieved from the Internet <URL:http://dx.doi.org/10.1158/2159-8290.CD-14-1029> DOI: 10.1158/2159-8290.CD-14-1029

## Description

### Technical Field

The present disclosure relates to compounds and pharmaceutical compositions thereof as FGFR4 kinase inhibitors and use of said compounds and compositions in the treatment of FGFR4-mediated diseases.

### Background Art

FGFR (fibroblast growth factor receptor), as a family of receptor tyrosine kinases, includes FGFR1, FGFR2, FGFR3, and FGFR4, and consists of an extracellular variant region, a conserved region that binds heparan sulfate proteoglycan, an FGF binding region, a single transmembrane region, and an intracellular tyrosine kinase region. Most FGFs form complexes with FGFRs and heparin under the assistance of a co-receptor Klotho, resulting in autophosphorylation of the conformationally altered FGFR intracellular kinase domain to activate the STATS signaling pathway. Autophosphorylated FGFR can also phosphorylate its aptamer protein FRS2α and activate the Grb2/Sos1 complex to initiate downstream MAPK and PI3K/AKT signaling pathways. In addition, FGFR activates phospholipase C-y (PLC-y), phosphorylates RAF, strengthens MAPK signaling, and plays a role in regulating cell proliferation, differentiation, and migration in an FRS2α-independent manner. MAPK signaling pathway is mainly associated with FGFR-mediated cell proliferation and metastasis, while PI3K/AKT signaling pathway is mainly associated with cell motility and survival. The FGFR4 signaling pathway is controlled strictly under physiological conditions and deregulated FGFR4 signaling leads to the development, proliferation, survival, and metastasis of cancer.

About 30% of patients with hepatocellular carcinoma have abnormally activated FGFR4 in their tumors. FGFR4 inhibitors showed superior potential for the treatment of HCC in both preclinical and clinical trials and had good safety and a sufficient toxicity/effectiveness window.

Small molecule tyrosine kinase inhibitors block cell proliferation signals by blocking the binding activity of intracellular kinases to ATP. Small molecule inhibitors of FGFR4 can be divided into pan-FGFR and FGFR4 specific small molecule inhibitors. Due to the similar structure of the kinase domains of FGFR1, FGFR2, and FGFR3, the inhibitors developed at this stage against these three kinases have similar effects. However, the FGFR4 kinase domain differs from the FGFR1-3 kinase domains to a certain extent, and therefore many inhibitors that can effectively inhibit FGFR1-3 have poor effects on FGFR4. For example, small molecule inhibitors entering Phase I or Phase II clinical trial such as CH5183284, BGJ398, and AZD4547 were more selective for FGFR1-3 (IC₅₀<10 nmol/L) than for FGFR4. JNJ-42756493 and LY2874455 are a few pan FGFR small-molecule inhibitors with equally high-efficiency inhibition effect on FGFR1-4, the IC50 of which reaches a single-digit nanomolar level. By inhibiting the FGF/FGFR signaling pathway, both JNJ-42756493 and LY2874455 showed FGFR-dependent anti-proliferation effects in cells, and had a strong inhibitory effect on transplanted tumors with abnormal FGFR, and showed a dose-response to inhibit tumor growth. The results of Phase I clinical trial of JNJ-42756493 (NCT01962532) determined that the drug dose for Phase II clinical trial (RP2D) was 10 mg/day (drug administration for 7 days, drug withdrawal for 7 days). The results of Phase I clinical trial of LY2874455 (NCT 01212107) determined that its RP2D was 16 mg/day, twice a day. In Phase I clinical trial of AZD4547 (NCT00979134), AZD4547 showed strong tumor-killing activity in patients with FGFR gene-amplified squamous non-small cell lung cancer and was well tolerated at a dose of 80 mg. The Phase II/III clinical trial (NCT01761747) of Ponatinib in the treatment of patients with advanced squamous cell lung carcinoma with abnormal FGFR was terminated by adverse reactions.

WO2018004258A discloses a heterocyclic derivative compound and a use thereof. WO2018004258A further discloses a novel heterocyclic detivative compound having selective inhibitory activity against a fibroblast growth factor receptor (FGFR), and a pharmaceutical composition comprising the same for preventing or treating various diseases associated with the FGFR.

WO2014144737A discloses compouds useful as inhibitors of protein kinases, containing a cysteine residue in the ATP binding site. WO2014144737A further discloses pharmaceutically acceptable compositions comprising therapeutically effective amounts of one or more of the proteim kinase inhibitor compounds and methods of using said compositions in the treatmentof cancers and carcinomas.

WO2017050864A discloses quinoxaline, quinoline and quinazolinone derivative compounds, pharmaceutical compositions comprising said compounds, processes for the preparation of said compounds and the use of said compounds in the treatment of diseases,e.g. cancer.

WO2018113584A discloses an FGFR4 inhibitor having the structure of formula (I) and a preparation method therefor and the use thereof.

WO2014011900A discloses imhibitors of FGFR, pharmaceutical compositions including such compounds, and methods of using such compounds and compositions to inhibit the activity of tyrosine kinases.

WO2018153373A discloses an azatricyclic compound (as represented by formula 1) which acts as an inhibitor of fibroblast growth factor receptors (FGFR), as well as a pharnaceutical composition thereof, a preparation method, and a use therefore in the treatment of FGFR-ediated diseases.

"First Selective Small Molecule Inhibitor of FGFR4 for the Treatment of Hepatocellular Carcinomas with an Activated FGFR4 Signaling Pathway" discloses that aberrant signaling through the fibroblast growth factor 19 (FGF19)/fibroblast growth factor receptor 4 (FGFR4) signaling complex has been shown to cause hepatocellular carcinoma (HCC) in mice and has been implicated to paly a similar role in humans.

A lack of selective FGFR kinase inhibitors results in hyperphosphatemia, onycholysis, alopecia, mucositis, dysgeusia, and mucosal dryness, conjunctivitis, keratitis, sears optical, asymptomatic retinal pigment layer detachment, osteoarticular pain, myalgia, and other adverse reactions due to off-target, limiting their clinical use.

The compound disclosed by the present disclosure is an FGFR4 protein kinase inhibitor which can inhibit FGFR4 tyrosine kinase with high selectivity, and has a weak inhibition effect on FGFR1-3, to safely and effectively treat liver cancer patients with high FGFR4 expression.

### Summary of the Invention

In one aspect, disclosed are the compounds below, or a pharmaceutically acceptable salt, solvate, polymorph or tautomer thereof (the compounds marked with * are not part of the claimed invention): 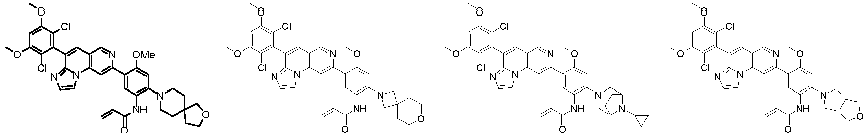 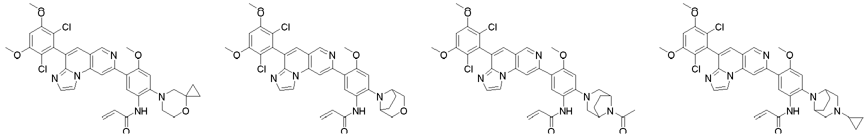 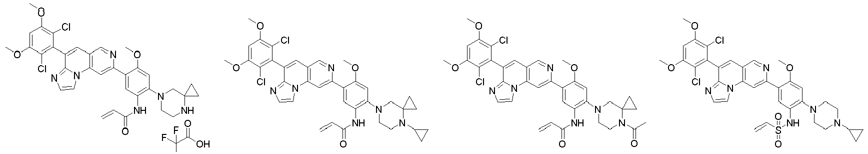 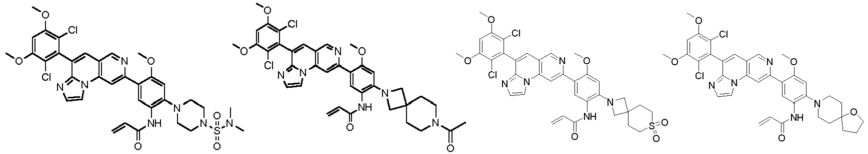 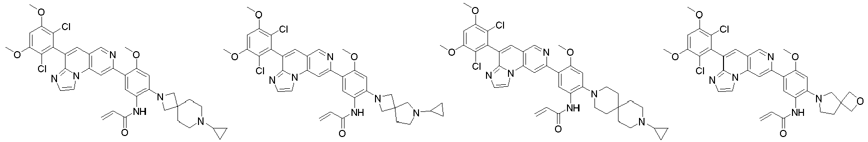 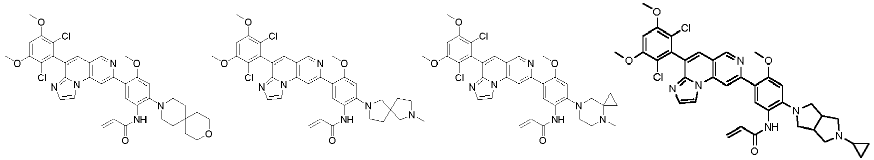 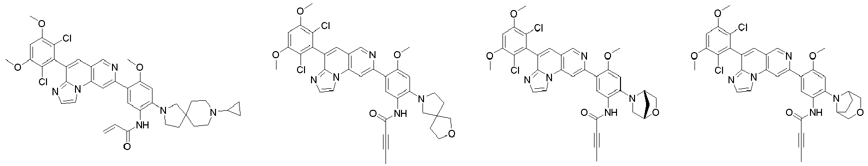 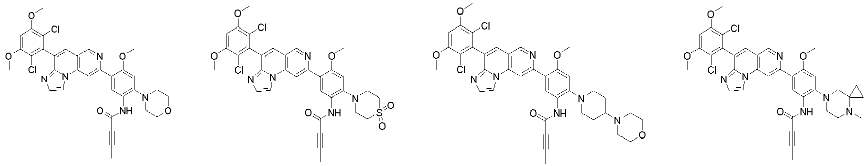 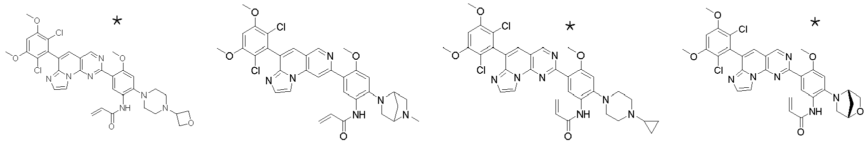 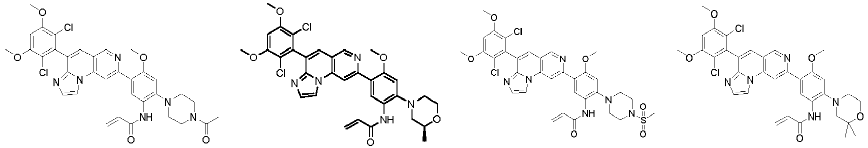 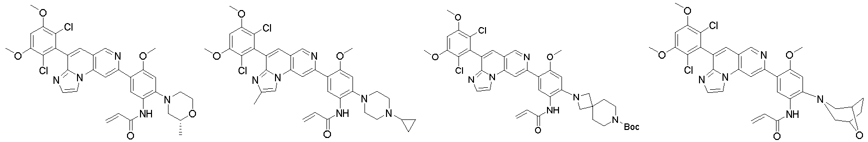 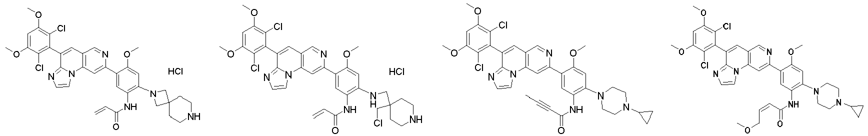 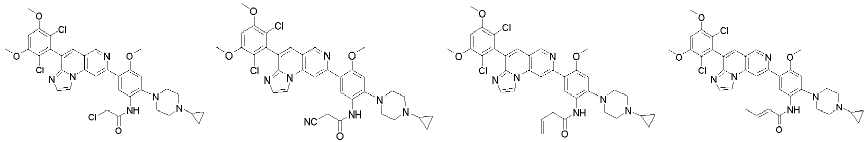 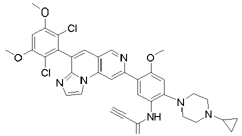 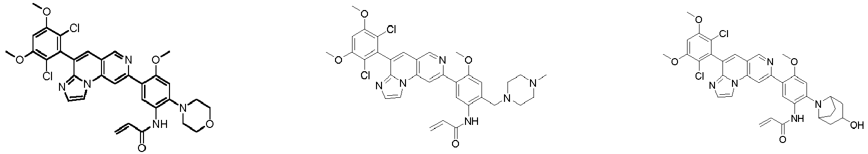 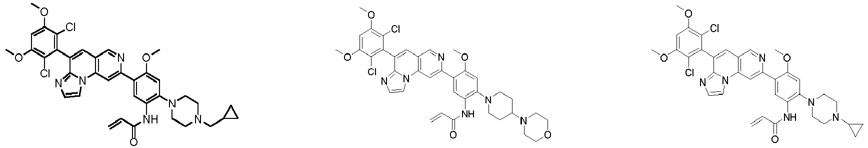 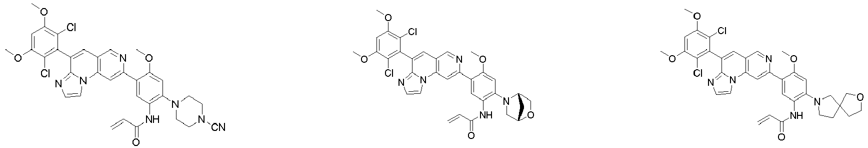 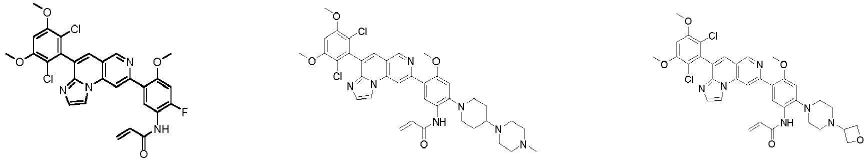 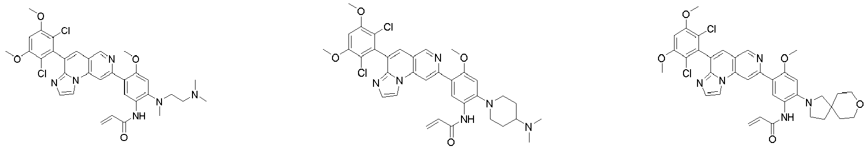 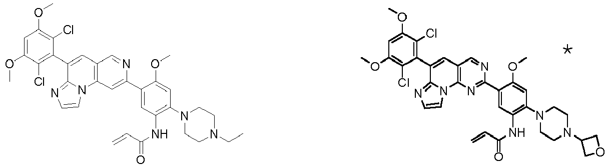 or a pharmaceutically acceptable salt, solvate, polymorph or tautomer thereof.

Compounds as recited above and in the claims of the invention can for use in the treatment of an FGFR4-mediated disease; In some embodiments, said FGFR4-mediated disease is non-small cell lung cancer, gastric carcinoma, multiple myeloma, liver cancer, cholangiocarcinoma, prostate cancer, skin cancer, ovarian cancer, breast cancer, colon cancer, glioma, and rhabdomyosarcoma, preferably liver cancer and cholangiocarcinoma.

Another aspect of the invention also relates to a pharmaceutical composition comprising the above compounds of the invention or a pharmaceutically acceptable salt, solvate, polymorph or tautomer thereof, and a pharmaceutically acceptable carrier.

In another aspect, disclosed are the compounds as recited above and in the claims or a pharmaceutically acceptable salt, solvate, polymorph or tautomer thereof, or the pharmaceutical composition above for use in the treatment of an FGFR4-mediated disease, comprising administering to an object an effective amount of a compound of Formula I or II or a pharmaceutically acceptable salt, solvate, polymorph or tautomer thereof, or the pharmaceutical composition above; In some embodiments, said FGFR4-mediated disease is non-small cell lung cancer, gastric carcinoma, multiple myeloma, liver cancer, cholangiocarcinoma, prostate cancer, skin cancer, ovarian cancer, breast cancer, colon cancer, glioma, and rhabdomyosarcoma, preferably liver cancer and cholangiocarcinoma.

In some embodiments of the present invention, the object involved in the present invention is a mammal including humans.

In another aspect, disclosed is a compound of the invention or a pharmaceutically acceptable salt, solvate, polymorph or tautomer thereof in the manufacture of a medicament for the treatment of an FGFR4-mediated disease; In some embodiments, said FGFR4-mediated disease is non-small cell lung cancer, gastric carcinoma, multiple myeloma, liver cancer, cholangiocarcinoma, prostate cancer, skin cancer, ovarian cancer, breast cancer, colon cancer, glioma, and rhabdomyosarcoma, preferably liver cancer and cholangiocarcinoma.

### Detailed Description

Exemplary embodiments utilizing the principles of the present invention are set forth in the following detailed description. The features and advantages of the present invention may be better understood with reference to the following contents of the present invention.

It is to be understood that the scope of the various aspects of the present invention is determined by the scope of the appended claims.

Unless defined otherwise, all technical and scientific terms used herein have the same meaning as commonly understood by one of the ordinary skills in the art.

It is to be understood that both the foregoing general description and the following detailed description are exemplary and explanatory and are not restrictive of any inventive subject matter. The use of the singular also includes the plural unless specifically stated otherwise. The use of "or" means "and/or" unless otherwise indicated. Furthermore, the use of the term "include" and other forms, such as "comprise", "have", and "contain", are not restrictive.

### Some chemical terms

The term "option", "optional" or "optionally" means that the event or situation described later may or may not occur, and the description includes the occurrence of the event or situation and the non-occurrence of the event or situation. For example, "optionally substituted alkyl" means "unsubstituted alkyl" or "substituted alkyl". Also, an optionally substituted group may be unsubstituted (for example: -CH₂CH₃), completely substituted (for example: -CF₂CF₃), monosubstituted (for example: -CH₂CH₂F) or any level between mono- and completely substituted (e.g: -CH₂CHF₂, -CF₂CH₃, -CFHCHF₂, etc.). Those skilled in the art can understand that for any group containing one or more substituents, any substitution or substitution pattern that is impossible to exist in space and/or cannot be synthesized will not be introduced.

Unless otherwise indicated, conventional methods within the skill of the art are employed, such as mass spectrometry, nuclear magnetic resonance, high-performance liquid chromatography, infrared and ultraviolet/visible spectroscopy, and pharmacological methods. Unless specifically defined otherwise, the relevant terms and experimental procedures and techniques in analytical chemistry, organic synthetic chemistry, and pharmaceutical and medicinal chemistry herein are known in the art. Standard techniques may be used in chemical synthesis, chemical analysis, pharmaceutical preparation, formulation and delivery, and treatment of patients. For example, reactions and purifications can be carried out using the manufacturer's instructions for the kit, or in a manner well known in the art or under the instructions of the present invention. The techniques and methods described above can generally be implemented according to conventional methods well known in the art, as described in various general and more specific documents cited and discussed in this specification. In the present specification, groups and substituents thereof may be selected by those skilled in the art to provide stable moieties and compounds.

When a substituent is described by a conventional chemical formula written from left to right, the substituent also includes chemically equivalent substituents obtained when the structural formula is written from right to left. For example, -CH₂O- is equivalent to -OCH₂-.

As used herein, the terms "group" and "chemical group" refer to a particular moiety or functional group of a molecule. Chemical groups are often considered to be chemical entities embedded or attached to a molecule.

Some of the chemical groups named herein may use abbreviated notation to indicate the total number of carbon atoms. For example, C₁-C₆ alkyl describes an alkyl group, as defined below, having a total of 1 to 6 carbon atoms. The total number of carbon atoms indicated in the abbreviated notation does not include the carbon atoms on the possible substituents.

The term "halogen", "halo" or "halide" refers to bromine, chlorine, fluorine, or iodine.

As used herein, the terms "aroma", "aromatic ring", "aromatic", "aromaticity", and "aromatic ring" refer to a planar ring portion of one or more rings having a delocalized electron conjugation system containing 4n+2 electrons, where n is an integer. The aromatic ring may be formed from 5, 6, 7, 8, 9, or more atoms. The aromatic compound may be optionally substituted and may be monocyclic or fused polycyclic. The term aromatic compounds include all carbocyclic rings (e.g., benzene rings) and rings containing one or more heteroatoms (e.g., pyridine).

The term "heteroatom" or "hetero" as used herein alone or as part of another ingredient refers to an atom other than carbon and hydrogen. The heteroatoms are independently selected from oxygen, nitrogen, sulfur, phosphorus, silicon, selenium, and stannum, but are not limited to these atoms. In embodiments where two or more heteroatoms are present, the two or more heteroatoms may be the same as each other, or some or all of the two or more heteroatoms may be different from each other.

The terms "fused" or "fused ring" as used herein, alone or in combination, refer to a cyclic structure in which two or more rings share one or more bonds.

The terms "spiro" or "spirocyclic", as used herein, alone or in combination, refer to a cyclic structure in which two or more rings share one or more atoms.

The term "alkyl" as used herein alone or as part of other components (such as monoalkylamino) refers to an optionally substituted linear or optionally substituted branched monovalent saturated hydrocarbon having 1 to 12 carbon atoms, preferably 1 to 8 carbon atoms, more preferably 1 to 6 carbon atoms, attached to the rest of the molecule via a single bond, for example, methyl, ethyl, n-propyl, isopropyl, n-butyl, isobutyl, sec-butyl, tert-butyl, n-pentyl, n-hexyl, n-heptyl, 2-methylhexyl, 3-methylhexyl, n-octyl, n-nonyl, n-decyl.

The term "alkylene", as used herein, alone or in combination, refers to a divalent group derived from a monovalent alkyl group as defined above. Examples include, but are not limited to, methylene (-CH₂), ethylidene (-CH₂CH₂), propylidene (-CH₂CH₂CH₂), and isopropylidene (-CH(CH₃)CH₂).

The term "cycloalkyl" as used herein alone or as part of another component refers to a stable monovalent non-aromatic monocyclic or polycyclic hydrocarbon group containing only carbon and hydrogen atoms, possibly including fused, spiro, or bridged ring systems, containing from 3 to 15 ring-forming carbon atoms, preferably from 3 to 10 ring-forming carbon atoms, more preferably from 3 to 8 ring-forming carbon atoms, which may be saturated or unsaturated, attached to the rest of the molecule via a single bond. Non-limiting examples of "cycloalkyl" include, but are not limited to, cyclopropyl, cyclobutyl, cyclopentyl, cyclohexyl, cycloheptyl.

The terms "heterocyclyl", "heterocycloalkyl", "heterocycle" as used herein alone or as part of another component, refer to a stable 3-18 membered monovalent non-aromatic ring containing from 2 to 12 carbon atoms and from 1 to 6 heteroatoms selected from nitrogen, oxygen, and sulfur. Unless otherwise specified, a heterocyclyl group may be a monocyclic, bicyclic, tricyclic, or tetracyclic ring system, which may contain fused, spiro, or bridged ring systems, nitrogen, carbon, or sulfur on the heterocyclyl may be optionally oxidized, the nitrogen atom may be optionally quaternized, and the heterocyclyl may be partially or completely saturated. The heterocyclyl may be attached to the rest of the molecule via a single bond through a carbon atom or heteroatom on the ring. A heterocyclyl containing fused rings may contain one or more aromatic or heteroaromatic rings as long as the atoms on the non-aromatic ring are attached to the rest of the molecule. For purposes of this application, the heterocyclyl is preferably a stable 4-11 membered monovalent non-aromatic monocyclic or bicyclic ring containing from 1 to 3 heteroatoms selected from nitrogen, oxygen and sulfur, more preferably a stable 4-8 membered monovalent non-aromatic monocyclic ring containing from 1 to 3 heteroatoms selected from nitrogen, oxygen, and sulfur. Non-limiting examples of heterocyclyl include azepanyl, azetidinyl, decahydroisoquinolinyl, dihydrofuranyl, indolinyl, dioxolanyl, 1,1-dioxo-thiomorpholinyl, imidazolidinyl, imidazolinyl, isothiazolidinyl, isoxazolidinyl, morpholinyl, octahydroindolyl, octahydroisoindolyl, oxazinyl, piperazinyl, piperidinyl, 4-piperidonyl, pyranyl, pyrazolidinyl, pyrrolidinyl, quinolizinyl, quinuclidinyl, tetrahydrofuranyl, tetrahydropyranyl.

The terms "aromatic ring", "aromatic ring group", "aromatic group", "aryl", or the prefix "ar" as used herein alone or as part of another component, (such as in "aralkyl"), refer to a hydrocarbon ring system containing hydrogen, 6 to 18 ring-forming carbon atoms, preferably 6 to 10 ring-forming carbon atoms, and at least one aromatic ring. For purposes of the present disclosure, an aromatic ring group may be a monocyclic, bicyclic, tricyclic or tetracyclic ring system, which may contain fused or bridged ring systems. The aryl carbon atom may be attached to the rest of the molecule via a single bond. Non-limiting examples of aryl groups include phenyl, naphthyl, anthracenyl, phenanthrenyl, fluorenyl. In the present disclosure, the aryl group is preferably a C₆-C₁₀ aryl group, more preferably a phenyl group.

The term "heteroaryl" as used herein alone or as part of another component refers to a 5-16 membered ring system containing from 1 to 15 carbon atoms, preferably from 1 to 10 carbon atoms, from 1 to 4 heteroatoms selected from nitrogen, oxygen and sulfur, and at least one aromatic ring. Unless otherwise specified, a heteroaryl may be a monocyclic, bicyclic, tricyclic, or tetracyclic ring system, which may contain fused or bridged ring systems, so long as the point of attachment to the rest of the molecule is an aromatic ring atom. The nitrogen, carbon, and sulfur atoms on the heteroaromatic ring may be optionally oxidized and the nitrogen atom may be optionally quaternized. For the purposes of the present disclosure, heteroaryl is preferably a stable 4-11 membered monoaromatic ring containing from 1 to 3 heteroatoms selected from nitrogen, oxygen, and sulfur, more preferably a stable 5-8 membered monoaromatic ring containing from 1 to 3 heteroatoms selected from nitrogen, oxygen, and sulfur. Non-limiting examples of heteroaryl groups include acridinyl, azepinyl, benzimidazolyl, benzindolyl, benzodioxinyl, benzodioxolyl, benzofuranonyl, benzofuranyl, benzonaphthofuranyl, benzopyranonyl, benzopyranyl, benzopyrazolyl, benzothiadiazolyl, benzothiazolyl, benzotriazolyl, furanyl, imidazolyl, indazolyl, indolyl, oxazolyl, purinyl, pyrazinyl, pyrazolyl, pyridazinyl, pyridinyl, pyrimidinyl, pyrrolyl, quinazolinyl, quinolinyl, quininyl, tetrazolyl, thiadiazolyl, thiazolyl, thienyl, triazinyl, triazolyl. As used herein, heteroaryl is preferably a 5-8 membered heteroaryl comprising from 1 to 3 heteroatoms selected from nitrogen, oxygen, and sulfur, more preferably pyridinyl, pyrimidinyl, and thiazolyl.

As used herein, the term "polymorph" or " polymorphism" refers to a compound of the present invention having a variety of lattice morphologies. Some of the compounds of the present invention may have more than one crystal form, and the present invention encompasses all polymorphs or mixtures thereof.

Intermediates of the compounds of the present disclosure and polymorphs thereof are also within the scope of the present disclosure (but not of the claimed invention).

Unless otherwise specified, the olefinic double bond contained in the compound of the present invention includes E and Z isomers.

It is to be understood that the compounds of the present invention may contain asymmetric centers. These asymmetric centers may independently be in the *R* or S configuration. Some of the compounds of the present invention may also show cis-trans isomerism, which is obvious to those skilled in the art. It is to be understood that the compounds of the present invention include individual geometric and stereoisomers thereof as well as mixtures thereof, including racemic mixtures. These isomers may be separated from their mixtures by practicing or modifying known methods, such as chromatographic techniques and recrystallization techniques, or they may be prepared separately from the appropriate isomers of their intermediates.

As used herein, the term "pharmaceutically acceptable salt" includes both acid and alkali addition salts.

"Pharmaceutically acceptable acid addition salts" refer to those salts formed with inorganic acids such as, but not limited to, hydrochloric acid, hydrobromic acid, sulfuric acid, nitric acid, and phosphoric acid; or with organic acids such as, but not limited to, acetic acid, 2,2-dichloroacetic acid, adipic acid, alginic acid, ascorbic acid, aspartic acid, benzenesulfonic, benzoic acid, capric acid caproic acid, carbonic acid, cinnamic acid, and citric acids, which retain the biological effectiveness and properties of the free alkali of the compound, and are not biologically or otherwise undesirable. "Pharmaceutically acceptable alkali addition salts" refers to those salts that retain the biological effectiveness and properties of the free acids of the compounds and are not biologically or otherwise undesirable. These salts are prepared by reacting free acids with inorganic or organic alkalis. Salts formed by reaction with inorganic alkalis include, but are not limited to, sodium, potassium, lithium, ammonium, calcium, magnesium, iron, zinc, copper, manganese, aluminum salts, and the like. Preferred inorganic salts are ammonium, sodium, potassium, calcium, and manganese salts.

Organic alkalis that form salts include, but are not limited to, primary, secondary, tertiary, and cyclic amines, such as ammonia, isopropylamine, trimethylamine, diethylamine, triethylamine, tripropylamine, ethanolamine, diethanolamine, ethanolamine, dicyclohexylamine, ethylenediamine, purine, piperazine, piperidine, choline, and caffeine. Particularly preferred organic alkalis include isopropylamine, diethylamine, ethanolamine, trimethylamine, dicyclohexylamine, choline, and caffeine.

Crystallization often produces solvates of the compounds of the present invention. The term "solvate" as used herein refers to a complex composed of one or more molecules of the compound of the present invention and one or more solvent molecules.

The solvent may be water, in which case the solvate is a hydrate. It may additionally be an organic solvent. Thus, the compounds of the present invention may exist as hydrates, including monohydrate, dihydrate, hemihydrate, trihydrate, and tetrahydrate, as well as the corresponding solvated forms. The compounds of the present invention may be true solvates, but in other cases, the compounds of the present invention may simply accidentally retain water or a mixture of water with some other solvent. The compounds of the present invention may be reacted in a solvent or precipitated or crystallized in a solvent. Solvates of the compounds of the present invention are also included in the scope of the present invention.

The term "pharmaceutical composition" as used herein refers to a formulation incorporating a compound of the present invention and a medium generally accepted in the art for delivering a biologically active compound to a mammal, such as a human. Such media comprise all pharmaceutically acceptable carriers.

The term "acceptable" as used herein in connection with a formulation, composition, or ingredient means there is no sustained deleterious effect on the overall health of the subject being treated.

The term "pharmaceutically acceptable" as used herein refers to a substance (e.g., a carrier or diluent) that does not affect the biological activity or properties of the compounds of the present invention, and is relatively nontoxic, i.e., the substance can be administered to an individual without causing an undesirable biological response or interaction in an undesirable manner with any of the components contained in the composition.

"Pharmaceutically acceptable carrier" includes, but is not limited to, adjuvants, carriers, excipients, auxiliaries, deodorants, diluents, preservatives, dyes/colorants, flavor enhancers, surfactants and wetting agents, dispersing agents, suspending agents, stabilizers, isotonic agents, solvents, or emulsifying agents, which may be used in humans and domesticated animals, as approved by the relevant government administration.

As used herein, the terms "subject", "patient, "object", or "individual" refers to an individual suffering from a disease, disorder, or condition, etc., including mammals and non-mammals. Examples of mammals include, but are not limited to, any member of the class Mammalia: humans, non-human primates (e.g., chimpanzees and other apes and monkeys); livestock, such as cattle, horses, sheep, goats, pigs; domestic animals such as rabbits, dogs, and cats; laboratory animals, including rodents, such as rats, mice, and guinea pigs, etc. Non-mammals include, but are not limited to, birds, and fish. In an embodiment related to methods and compositions provided herein, the mammal is a human.

The term "treatment" as used herein refers to the treatment of a disease or condition associated with a mammal, particularly a human, including
(i) preventing a mammal, particularly a mammal that has previously been exposed to a disease or condition but has not yet been diagnosed with the disease or condition, from developing the corresponding disease or condition;
(ii) inhibiting the disease or disorder, i.e., controlling its development;
(iii) alleviating the disease or disorder, i.e., causing regression of the disease or disorder; and
(iv) alleviating symptoms caused by the disease or disorder.

The terms "disease" and "condition" as used herein may be used interchangeably and may have different meanings, as some specific diseases or conditions have no known pathogenic factors (so the cause of the disease remains unknown), and therefore they cannot be considered a disease but can only be considered an unwanted condition or syndrome, with more or less specific symptoms having been confirmed by clinical researchers.

As used herein, the terms "effective amount", "therapeutically effective amount", or "pharmaceutically effective amount" refer to an amount of at least one agent or compound that, upon administration, is sufficient to relieve to some extent one or more symptoms of the disease or disorder being treated. The result may be reduction and/or alleviation of signs, symptoms, or causes, or any other desired change in the biological system. For example, an "effective amount" for therapy is the amount of a composition comprising a compound disclosed herein required to provide a clinically significant disease remission effect. Effective amounts suitable for use in any individual case may be determined using techniques such as dose escalation test.

The terms "taking", "administration", "administering", etc., as used herein, refer to a method of delivering a compound or composition to the desired site for biological action. These methods include, but are not limited to, oral routes, duodenal routes, parenteral injection (including intravenous, subcutaneous, intraperitoneal, intramuscular, intraarterial injection or infusion), topical administration, and rectal administration. In preferred embodiments, the compounds and compositions discussed herein are administered orally.

### Preparation of compound of the invention

The following non-limiting examples are merely illustrative and do not limit the present invention in any way.

Unless otherwise stated, temperatures are in degrees Celsius. Reagents were purchased from commercial suppliers such as Sinopharm Chemical Reagent Beijing Co., Ltd., Alfa Aesar, or Beijing J&K Scientific Ltd., and these reagents were used without further purification unless otherwise specified.

Unless otherwise stated, the following reactions were carried out at room temperature, in an anhydrous solvent, under a positive pressure of nitrogen or argon, or using a drying tube; glassware was baked and/or dried by drying.

Unless otherwise stated, silica gel for column chromatography was supplied from Qingdao Haiyang Chemical Plant in 200-300 mesh size; thin layer chromatography silica gel precast slab (HSGF254) for preparative Thin Layer Chromatography was produced by Yantai Chemical Industry Research Institute; Thermo LCQ Fleet type (ESI) Liquid Chromatograph Mass Spectrometer was used for MS assay.

Nuclear magnetic data (¹H NMR) were run at 400 MHz using a Varian apparatus. The solvents used for nuclear magnetic data are CDCl₃, CD₃OD, D₂O, DMSO-d6, etc., based on tetramethylsilane (0.00 ppm) or based on the residual solvent (CDCl₃: 7.26 ppm; CD₃OD: 3.31 ppm; D₂O: 4.79 ppm; d6- DMSO: 2.50 ppm). When peak shape diversity is indicated, the following abbreviations denote different peak shapes: s (singlet), d (doublet), t (triplet), q (quartet), m (multiplet), br (broad), dd (doublet of doublets), dt (doublet of triplets). If a coupling constant is given, it is in Hertz (Hz).

### Detailed Description of the Invention

Exemplary embodiments of the present invention are provided in the following examples. The following examples are given by way of example only and are intended to assist ordinarily skilled in the art in the use of the present invention. The examples are not intended to limit the scope of the present invention in any way.

### Example 1

### N-(5-(4-(2,6-dichloro-3,5-dimethoxyphenyl)imidazo[1,2-a][1,6]naphthyridin-8-yl)-4-methoxy-2-(2-oxa-8-azaspiro[4.5]decan-8-yl)phenyl)acrylamide

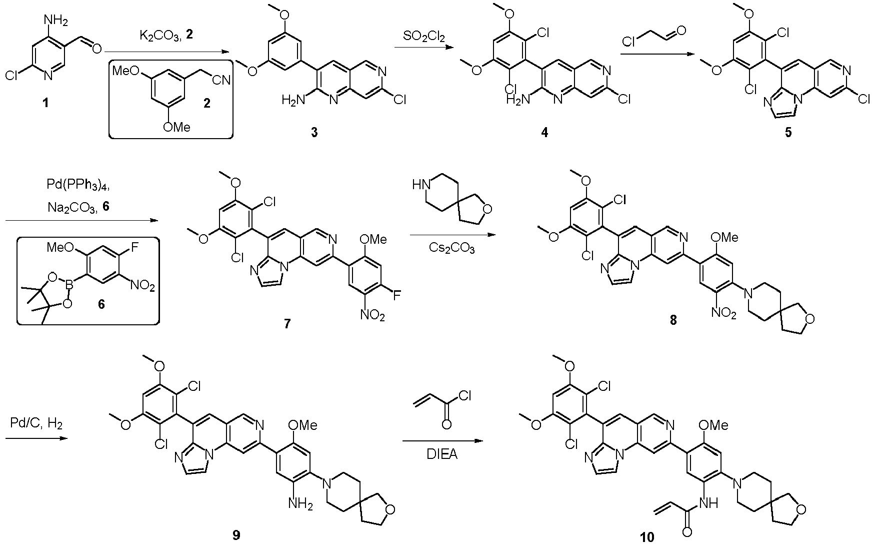

### Step 1: synthesis of compound 3

To a solution of compound **1** (2 g) in 1-Methyl-2-pyrrolidinone (15 mL) were added compound **2** (2.49 g) and potassium carbonate (5.29 g). The reaction solution was heated to 100 °C and reacted overnight. The reaction solution was cooled to room temperature and poured into water. The mixture was filtered, and the filter cake was washed with distilled water and dried to obtain compound **3** (3.58 g).

### Step 2: synthesis of compound 4

At -20 °C, to a solution of compound **3** (3.58 g) in THF (100 mL) was dropwise added sulfuryl chloride (2.3 mL). The reaction solution was stirred at this temperature for 2 hours. An aqueous solution of saturated sodium bicarbonate was used to quench the reaction. The mixture was allowed to stay and partition, the organic phase was separated, and the aqueous phase was extracted with ethyl acetate. The organic phases were combined, dried over anhydrous sodium sulfate, and concentrated under reduced pressure. The residue thus obtained was purified through flash column chromatography (dichloromethane/methanol = 100/1) to obtain compound **4** (3.24 g).

### Step 3: synthesis of compound 5

To a solution of compound **4** (3.24 g) in ethanol (18 mL) was added chloroacetaldehyde (18 mL). The reaction solution was heated to 80 °C and reacted overnight. The reaction solution was poured into water and filtered. The solid thus obtained was dried to obtain compound **5** (3.1 g).

### Step 4: synthesis of compound 7

Under nitrogen protection, to a solution of compound **5** (1.23 g) in dioxane/water (20 mL/5 mL) were added tetrakis(triphenylphosphine)palladium (500 mg), compound **6** (1.5 g) and anhydrous sodium carbonate (1.5 g). The reaction solution was heated to 80 °C and reacted overnight. The reaction solution was cooled to room temperature and concentrated under reduced pressure, and the residue thus obtained was separated and purified through flash silica gel column chromatography (dichloromethane/methanol = 500/1) to obtain compound **7** (1.12 g).

### Step 5: synthesis of compound 8

To a solution of compound **7** (200 mg) in DMF (1 mL) were added anhydrous caesium carbonate (120 mg) and morpholine (0.1 mL), and the mixture was heated to 60 °C and reacted overnight. The reaction solution was cooled to room temperature and concentrated under reduced pressure. The residue thus obtained was separated and purified through flash silica gel column chromatography (dichloromethane/methanol = 100/1 to 50/1) to obtain compound **8** (120 mg).

### Step 6: synthesis of compound 9

To a solution of compound **8** (120 mg) in tetrahydrofuran (5 mL) was added palladium on carbon (50 mg), and the system was purged with hydrogen gas. The reaction system was reacted at room temperature overnight and filtered through celite. The filtrate was concentrated under reduced pressure to obtain compound **9** (71 mg).

### Step 7: synthesis of compound 10

In an ice bath, to a solution of compound **9** (71 mg) in dichloromethane (2 mL) were added DIEA (5 µL) and acryloyl chloride (11 µL). The reaction solution was stirred in an ice bath for 2 h. Methanol was added to quench the reaction, and the mixture was concentrated under reduced pressure. The residue thus obtained was separated and purified through preparative thin layer chromatography (dichloromethane/methanol =30/1) to obtain compound **10** (35 mg). ¹H NMR (400 MHz, CDCl₃), 9.21 (1H, s), 9.00 (1H, s), 8.35 (1H, s), 8.22 (1H, s), 8.12 (1H, d, *J=* 1.2 MHz), 7.69 (1H, d, *J* = 1.2 Hz), 7.50 (1H, s), 6.81 (1H, s), 6.70 (1H, s), 6.42 (1H, d, *J* = 16.8 Hz), 6.30 (1H, dd, *J* = 16.8 Hz, 10.0 Hz), 5.77 (1H, d, *J* = 10.0 Hz), 3.97 (6H, s), 3.91-3.95 (5H, m), 3.68 (2H, s), 2.85-2.99 (4H, m), 1.87 (2H, t, *J* = 7.2 Hz), 1.78-1.83 (4H, m).

### Example 2

### N-(5-(4-(2,6-dichloro-3,5-dimethoxyphenyl)imidazo[1,2-a][1,6]naphthyridin-8-y l)-4-methoxy-2-(7-oxa-2-azaspiro[3.5]nonan-2-yl)phenyl)acrylamide

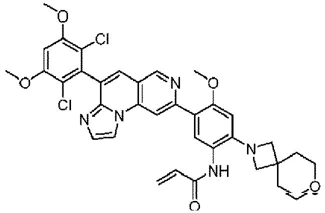

The synthetic method of example 2 was same as that of example 1. ¹H NMR (400 MHz, CDCl₃), 9.12 (1H, s), 8.41 (1H, s), 8.11 (1H, s), 8.01 (1H, s), 7.68 (1H, s), 7.46 (1H, s), 7.22 (1H, s), 6.70 (1H, s), 6.42 (1H, d, *J* = 16.8Hz), 6.31 (1H, dd, *J* = 16.8 Hz, 10.8 Hz), 6.09 (1H, s), 5.78 (1H, d, *J* = 10.8 Hz), 3.97 (6H, s), 3.94 (3H, s), 3.77 (4H, s), 3.60-3.67 (4H, m), 1.77-1.83 (4H, m).

### Example 3

### N-(2-(8-cyclopropyl-3,8-diazabicyclo[3.2.1]octan-3-yl)-5-(4-(2,6-dichloro-3,5-di methoxyphenyl)imidazo[1,2-a][1,6]naphthyridin-8-yl)-4-methoxyphenyl)acrylamide

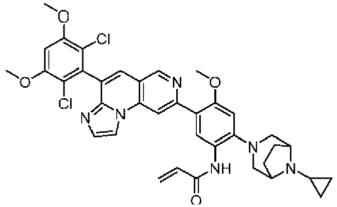

The synthetic method of example 3 was same as that of example 1. ¹H NMR (400 MHz, CDCl₃), 9.20 (1H, s), 8.95 (1H, s), 8.42 (1H, s), 8.37 (1H, s), 8.13 (1H, s), 7.69 (1H, s), 7.49 (1H, s), 7.02-7.13 (1H, brs), 6.70 (1H, s), 6.45 (1H, d, *J* = 16.8 Hz), 6.30 (1H, dd, *J* = 16.8 Hz, 10.0 Hz), 5.78 (1H, d, *J* = 10.0 Hz), 3.96 (6H, s), 3.88 (3H, s), 3.21-3.78 (4H, m), 2.79 (2H, d, *J* = 10.4 Hz), 2.20-2.38 (2H, m), 1.97-2.18 (3H, m), 0.81-0.91 (2H, m), 0.56-0.70 (2H, m).

### Example 4

### N-(5-(4-(2,6-dichloro-3,5-dimethoxyphenyl)imidazo[1,2-a][1,6]naphthyridin-8-y l)-4-methoxy-2-(tetrahydro-1H-furo[3,4-c]pyrrol-5(3H)-yl)phenyl)acrylamide

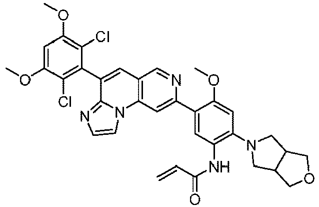

The synthetic method of example 4 was same as that of example 1. ¹H NMR (400 MHz, CDCl₃), 9.20 (1H, s), 8.87 (1H, s), 8.37 (1H, s), 8.15 (1H, s), 8.12 (1H, d, *J* = 1.6 Hz), 7.68 (1H, d, *J* = 1.6 Hz), 7.49 (1H, s), 6.79 (1H, s), 6.70 (1H, s), 6.43 (1H, d, *J* = 17.2 Hz), 6.30 (1H, dd, *J* = 17.2 Hz, 10.0 Hz), 5.76 (1H, d, *J* = 10.0 Hz), 3.88-4.01 (11H, m), 3.76 (2H, d, *J* = 10.0 Hz), 3.17-3.24 (2H, m), 2.96-3.07 (4H, m).

### Example 5

### N-(5-(4-(2,6-dichloro-3,5-dimethoxyphenyl)imidazo[1,2-a][1,6]naphthyridin-8-y l)-4-methoxy-2-(4-oxa-7-azaspiro[2.5]octan-7-yl)phenyl)acrylamide

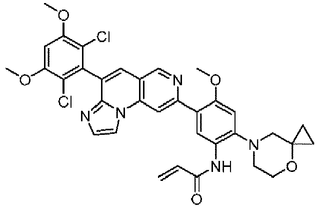

The synthetic method of example 5 was same as that of example 1. ¹H NMR (400 MHz, CDCl₃), 9.20 (1H, s), 9.01 (1H, s), 8.37 (2H, s), 8.12 (1H, s), 7.69 (1H, s), 7.49 (1H, s), 6.88 (1H, s), 6.70 (1H, s), 6.44 (1H, d, *J* = 16.8 Hz), 6.29 (1H, dd, *J* = 16.8 Hz, 10.8 Hz), 5.78 (1H, d, *J* = 10.8 Hz), 3.90-4.01 (11H, m), 3.10 (2H. t, *J* = 7.2 Hz), 2.87 (2H, s), 0.91-0.99 (2H, m), 0.58-0.66 (2H, m).

### Example 6

### N-(2-(3-oxa-8-azabicyclo[3.2.1]octan-8-yl)-5-(4-(2,6-dichloro-3,5-dimethoxyphe nyl)imidazo[1,2-a][1,6]naphthyridin-8-yl)-4-methoxyphenyl)acrylamide

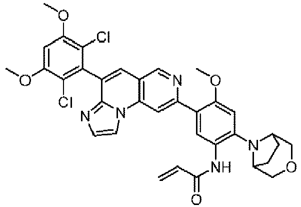

The synthetic method of example 6 was same as that of example 1. ¹H NMR (400 MHz, CDCl₃), 9.19 (1H, s), 8.85 (1H, s), 8.38 (1H, s), 8.12 (1H, s), 7.84 (1H, s), 7.68 (1H, s), 7.49 (1H, s), 6.70 (1H, s), 6.61 (1H, s), 6.43 (1H, d, *J* = 16.8 Hz), 6.29 (1H, dd, *J* = 16.8 Hz, 10.4 Hz), 5.78 (1H, d, *J* = 10.4 Hz), 3.88-3.98 (11H, m), 3.75 (2H, d, *J* = 10.8 Hz), 3.62-3.65 (2H, m), 2.01-2.18 (4H, m).

### Example 7

### N-(2-(8-acetyl-3,8-diazabicyclo[3.2.1]octan-3-yl)-5-(4-(2,6-dichloro-3,5-dimeth oxyphenyl)imidazo[1,2-a][1,6]naphthyridin-8-yl)-4-methoxyphenyl)acrylamide

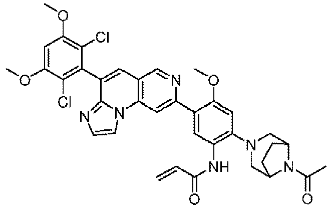

The synthetic method of example 7 was same as that of example 1. ¹H NMR (400 MHz, CDCl₃), 9.21 (1H, s), 8.99 (1H, s), 8.36 (1H, s), 8.32 (1H, s), 8.12 (1H, d, *J* = 1.6 Hz), 7.69 (1H, d, *J* = 1.6 Hz), 7.50 (1H, s), 6.86 (1H, s), 6.70 (1H, s), 6.46 (1H, d, *J* = 16.8 Hz), 6.28 (1H, dd, *J* = 16.8 Hz, 10.0 Hz), 5.80 (1H, d, *J* = 10.0 Hz), 4.85-4.90 (1H, m), 4.24-4.28 (1H, m), 3.97 (6H, s), 3.90 (3H, s), 3.21 (1H, d, *J* = 9.6 Hz), 2.93-3.01 (2H, m), 2.85(1H, d, *J* = 9.6 Hz), 1.98-2.28 (7H, m).

### Example 8

### N-(2-(3-cyclopropyl-3,8-diazabicyclo[3.2.1]octan-8-yl)-5-(4-(2,6-dichloro-3,5-di methoxyphenyl)imidazo[1,2-a][1,6]naphthyridin-8-yl)-4-methoxyphenyl)acrylamide

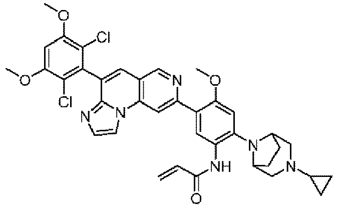

The synthetic method of example 8 was same as that of example 1. ¹H NMR (400 MHz, CDCl₃), 9.01 (1H, s), 8.41 (1H, s), 8.27 (1H, s), 8.09 (1H, s), 7.49 (1H, s), 7.40 (1H, s), 6.63 (1H, s), 6.51 (1H, s), 6.19-6.33 (2H, m), 5.67 (1H, d, *J* = 10.0 Hz), 3.97 (3H, s), 3.86 (6H, s), 3.57-3.63 (2H, m), 3.19-3.24 (1H, m), 2.76 (2H, d, *J* = 10.8 Hz), 2.54 (2H, d, *J* = 10.8 Hz), 1.68-1.83 (4H, m), 0.29-0.36 (2H, m), 0.19-0.25 (2H, m).

### Example 9

### N-(5-(4-(2,6-dichloro-3,5-dimethoxyphenyl)imidazo[1,2-a][1,6]naphthyridin-8-y l)-4-methoxy-2-(4,7-diazaspiro[2.5]octan-7-yl)phenyl)acrylamide trifluoroacetate

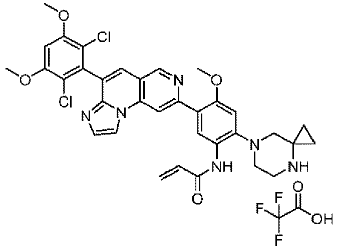

The synthetic method of example 9 was same as that of example 1. ¹H NMR (400 MHz, DMSO), 9.44 (2H, s), 9.32 (1H, s), 9.28 (1H, s), 8.79 (1H, s), 8.73 (1H, s), 8.34 (1H, s), 7.80 (1H, s), 7.68 (1H, s), 7.08 (1H, s), 6.88 (1H, s), 6.62 (1H, dd, *J* = 16.8 Hz, 10.0 Hz), 6.23 (1H, d, *J* = 16.8 Hz), 5.75 (1H, d, *J* = 10.0 Hz), 3.99 (6H, s), 3.96 (3H, s), 3.39-3.48 (2H, m), 3.23-3.32 (2H, m), 3.05 (2H, s), 1.06-1.12 (2H, m), 0.83-0.90 (2H, m).

### Example 10

### N-(2-(4-cyclopropyl-4,7-diazaspiro[2.5]octan-7-yl)-5-(4-(2,6-dichloro-3,5-dimet hoxyphenyl)imidazo[1,2-a][1,6]naphthyridin-8-yl)-4-methoxyphenyl)acrylamide

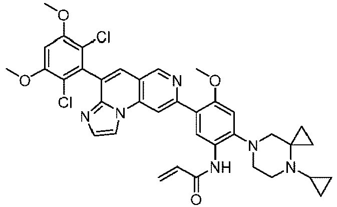

The synthetic method of example 10 was same as that of example 1. ¹H NMR (400 MHz, CDCl₃), 9.20 (1H, s), 8.99 (1H, s), 8.47 (1H, s), 8.35 (1H, s), 8.12 (1H, s), 7.69 (1H, s), 7.49 (1H, s), 6.90 (1H, s), 6.70 (1H, s), 6.43 (1H, d, *J* = 16.8 Hz), 6.29 (1H, dd, *J* = 16.8 Hz, 10.0 Hz), 5.78 (1H, d, *J* = 10.0 Hz), 3.97 (6H, s), 3.90 (3H, s), 3.02-3.21 (4H, m), 2.63-2.85 (2H, m), 1.95-2.15 (1H, m), 1.02-1.16 (2H, m), 0.40-0.61 (6H, m).

### Example 11

### N-(2-(4-acetyl-4,7-diazaspiro[2.5]octan-7-yl)-5-(4-(2,6-dichloro-3,5-dimethoxyp henyl)imidazo[1,2-a][1,6]naphthyridin-8-yl)-4-methoxyphenyl)acrylamide

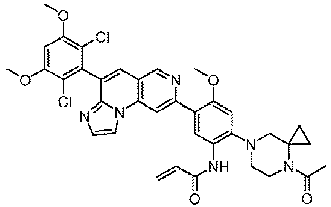

The synthetic method of example 11 was same as that of example 1. ¹H NMR (400 MHz, DMSO), 9.28 (1H, s), 9.22 (1H, s), 8.72 (1H, d, *J* = 1.2 Hz), 8.70 (1H, s), 8.32 (1H, s), 7.72 (1H, s), 7.60 (1H, d, *J* = 1.2 Hz), 7.07 (1H, s), 6.95 (1H, s), 6.57 (1H, dd, *J* = 16.8 Hz, 10.4 Hz), 6.23 (1H, dd, *J* = 16.8 Hz, 1.6 Hz), 5.75 (1H, d, *J* = 10.4 Hz), 3.67-4.05 (11H, m), 2.65-3.12 (4H, m), 2.13 (2.4 H, s), 2.03 (0.6 H, s), 0.78-1.06 (4H, m).

### Example 12

### N-(2-(4-cyclopropylpiperazin-1-yl)-5-(4-(2,6-dichloro-3,5-dimethoxyphenyl)imi dazo[1,2-a][1,6]naphthyridin-8-yl)-4-methoxyphenyl)ethenesulfonamide

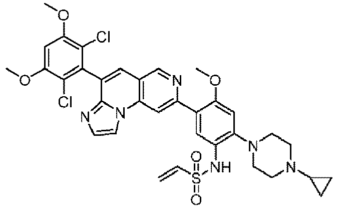

The synthetic method of example 12 was same as that of example 1. ¹H NMR (400 MHz, CDCl₃), 9.20 (1H, s), 8.39 (1H, s), 8.11 (2H, s), 7.70 (1H, s), 7.49 (1H, s), 7.40-7.47 (1H, brs), 6.93 (1H, s), 6.70 (1H, s), 6.66 (1H, dd, *J* = 16.4 Hz, 9.6 Hz), 6.42 (1H, d, *J* = 16.4 Hz), 6.01 (1H, d, *J* = 9.6 Hz), 3.98 (6H, s), 3.89 (3H, s), 3.63-3.69 (1H, m), 2.84-3.14 (4H, m), 1.66-1.92 (4H, m), 0.80-0.90 (2H, m), 0.56-0.66 (2H, m).

### Example 13

### N-(5-(4-(2,6-dichloro-3,5-dimethoxyphenyl)imidazo[1,2-a][1,6]naphthyridin-8-y l)-2-(4-(N,N-dimethylsulfamoyl)piperazin-1-yl)-4-methoxyphenyl)acrylamide

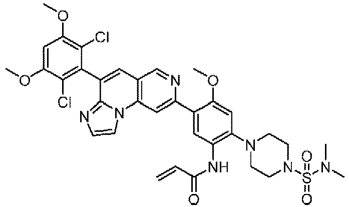

The synthetic method of example 13 was same as that of example 1. ¹H NMR (400 MHz, CDCl₃), 9.21 (1H, s), 8.99 (1H, s), 8.36 (1H, s), 8.12 (2H, s), 7.69 (1H, s), 7.50 (1H, s), 6.84 (1H, s), 6.70 (1H, s), 6.42 (1H, d, *J* = 16.8 Hz), 6.29 (1H, dd, *J* = 16.8 Hz, 10.4 Hz), 5.78 (1H, d, *J* = 10.4 Hz), 3.97 (6H, s), 3.92 (3H, s), 3.43-3.48 (4H, m), 3.01-3.06 (4H, m), 2.90 (6H, s).

### Example 14

### N-(2-(7-acetyl-2,7-diazaspiro[3.5]nonan-2-yl)-5-(4-(2,6-dichloro-3,5-dimethoxy phenyl)imidazo[1,2-a][1,6]naphthyridin-8-yl)-4-methoxyphenyl)acrylamide

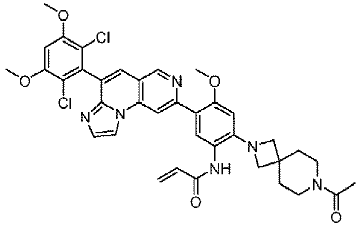

The synthetic method of example 14 was same as that of example 1. ¹H NMR (400 MHz, CDCl₃), 9.07 (1H, s), 8.39 (1H, s), 8.10 (1H, s), 7.95 (1H, s), 7.70-7.82 (1H, brs), 7.65 (1H, s), 7.43 (1H, s), 6.68 (1H, s), 6.36-6.44 (2H, m), 5.99-6.04 (1H, m), 5.70-5.75 (1H, m), 3.95 (6H, s), 3.90 (3H, s), 3.69-3.81 (4H, m), 3.48-3.55 (2H, m), 3.29-3.38 (2H, m), 2.06 (3H, s), 1.69-1.80 (4H, m).

### Example 15

### N-(5-(4-(2,6-dichloro-3,5-dimethoxyphenyl)imidazo[1,2-a][1,6]naphthyridin-8-y l)-2-(7,7-dioxido-7-thia-2-azaspiro[3.5]nonan-2-yl)-4-methoxyphenyl)acrylamide

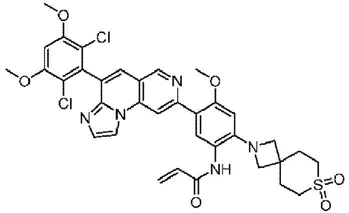

The synthetic method of example 15 was same as that of example 1. ¹H NMR (400 MHz, DMSO), 9.39 (1H, s), 9.20 (1H, s), 8.67 (1H, s), 8.66 (1H, s), 7.88 (1H, s), 7.66 (1H, s), 7.58 (1H, s), 7.06 (1H, s), 6.46 (1H, dd, *J* = 16.8 Hz, 10.4 Hz), 6.20 (1H, dd, *J* = 16.8 Hz, 1.6 Hz), 6.15 (1H, s), 5.69 (1H, dd, *J* = 10.4 Hz, 1.6 Hz), 3.98 (6H, s), 3.95 (3H, s), 3.80 (4H, s), 3.08-3.13 (4H, m), 2.17-2.23 (4H, m).

### Example 16

### N-(5-(4-(2,6-dichloro-3,5-dimethoxyphenyl)imidazo[1,2-a][1,6]naphthyridin-8-y l)-4-methoxy-2-(1-oxa-8-azaspiro[4.5]decan-8-yl)phenyl)acrylamide

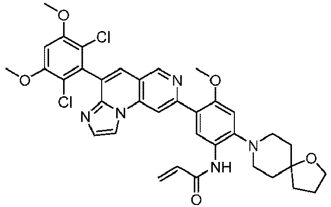

The synthetic method of example 16 was same as that of example 1. ¹H NMR (400 MHz, CDCl₃), 9.20 (1H, s), 8.99 (1H, s), 8.36 (1H, s), 8.31 (1H, s), 8.12 (1H, s), 7.68 (1H, s), 7.49 (1H, s), 6.90 (1H, s), 6.70 (1H, s), 6.41 (1H, d, *J* = 16.8 Hz), 6.28 (1H, dd, *J* = 16.8 Hz, 10.4 Hz), 5.75 (1H, d, *J* = 10.4 Hz), 3.97 (6H, s), 3.91 (2H, t, *J* = 6.8 Hz), 3.90 (3H, s), 3.07-3.13 (2H, m), 2.85-2.92 (2H, m), 1.99 (2H, qui, *J* = 6.8 Hz), 1.77-1.91 (6H, m).

### Example 17

### N-(2-(7-cyclopropyl-2,7-diazaspiro[3.5]nonan-2-yl)-5-(4-(2,6-dichloro-3,5-dimet hoxyphenyl)imidazo[1,2-a][1,6]naphthyridin-8-yl)-4-methoxyphenyl)acrylamide

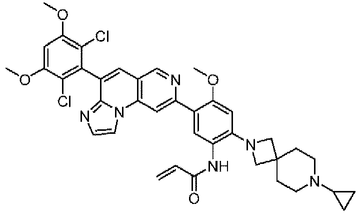

The synthetic method of example 17 was same as that of example 1. ¹H NMR (400 MHz, CDCl₃), 9.04 (1H, s), 9.38 (1H, s), 8.24 (1H, s), 8.09 (1H, s), 7.94 (1H, s), 7.63 (1H, s), 7.40 (1H, s), 6.67 (1H, s), 6.49 (1H, dd, *J* = 16.8 Hz, 10.0 Hz), 6.38 (1H, d, *J* = 16.8 Hz), 6.01 (1H, s), 5.70 (1H, d, *J* = 10.0 Hz), 3.93 (6H, s), 3.89 (3H, s), 3.74 (4H, s), 2.62-2.98 (4H, m), 1.89-2.06 (5H, m), 0.79-0.88 (2H, m), 0.55-0.64 (2H, m).

### Example 18

### N-(2-(6-cyclopropyl-2,6-diazaspiro[3.4]octan-2-yl)-5-(4-(2,6-dichloro-3,5-dimet hoxyphenyl)imidazo[1,2-a][1,6]naphthyridin-8-yl)-4-methoxyphenyl)acrylamide

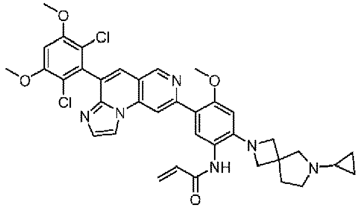

The synthetic method of example 18 was same as that of example 1. ¹H NMR (400 MHz, CDCl₃), 9.10 (1H, s), 8.41 (1H, s), 8.11 (1H, s), 7.99 (1H, s), 7.60-7.75 (2H, m), 7.44 (1H, S), 6.69 (1H, s), 6.38-6.45 (2H, m), 6.07 (1H, s), 5.74-5.77 (1H, m), 3.88-4.09 (11H, m), 2.92-3.24 (4H, m), 2.16-2.25 (2H, m), 1.88-2.06 (3H, m), 0.80-0.90 (2H, m), 0.53-0.62 (2H, m).

### Example 19

### N-(2-(9-cyclopropyl-3,9-diazaspiro[5.5]undecan-3-yl)-5-(4-(2,6-dichloro-3,5-di methoxyphenyl)imidazo[ 1,2-a][1,6]naphthyridin-8-yl)-4-methoxyphenyl)acrylamide

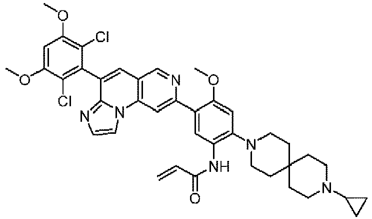

The synthetic method of example 19 was same as that of example 1. ¹H NMR (400 MHz, DMSO), 9.27 (1H, s), 9.09 (1H, s), 8.70 (1H, s), 8.69 (1H, s), 8.40 (1H, s), 7.70 (1H, s), 7.59 (1H, s), 7.06 (1H, s), 6.85 (1H, s), 6.65 (1H, dd, *J* = 16.8 Hz, 10.4 Hz), 6.22 (1H, d, *J* = 16.8 Hz), 5.71 (1H, d, *J* = 10.4 Hz), 3.98 (6H, s), 3.94 (3H, s), 2.88-2.96 (4H, m), 1.36-2.02 (13H, m), 0.96-1.12 (2H, m), 0.66-0.86 (2H, m).

### Example 20

### N-(5-(4-(2,6-dichloro-3,5-dimethoxyphenyl)imidazo[1,2-a][1,6]naphthyridin-8-y l)-4-methoxy-2-(2-oxa-6-azaspiro[3.4]octan-6-yl)phenyl)acrylamide

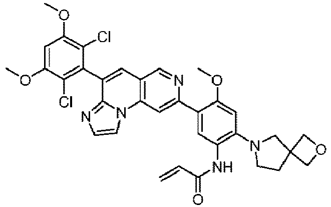

The synthetic method of example 20 was same as that of example 1. ¹H NMR (400 MHz, CDCl₃), 9.14 (1H, s), 8.41 (1H, s), 8.35 (1H, s), 8.11 (1H, s), 7.68 (1H, s), 7.47 (1H, s), 7.42 (1H, s), 6.70 (1H, s), 6.51 (1H, s), 6.42 (1H, d, *J* = 16.4 Hz), 6.29 (1H, dd, *J* = 16.4 Hz, 10.0 Hz), 5.77 (1H, d, *J* = 10.0 Hz), 4.64-4.71 (4H, m), 3.97 (6H, s), 3.94 (3H, s), 3.55 (2H, s), 3.33 (2H, t, *J* = 7.2 Hz), 2.27 (2H, t, *J* = 7.2 Hz).

### Example 21

### N-(5-(4-(2,6-dichloro-3,5-dimethoxyphenyl)imidazo[1,2-a][1,6]naphthyridin-8-y l)-4-methoxy-2-(3-oxa-9-azaspiro[5.5]undecan-9-yl)phenyl)acrylamide

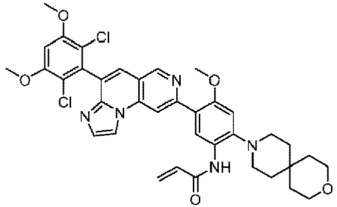

The synthetic method of example 21 was same as that of example 1. ¹H NMR (400 MHz, CDCl₃), 9.20 (1H, s), 8.99 (1H, s), 8.36 (1H, s), 8.21 (1H, s), 8.12 (1H, d, *J* = 1.6 Hz), 7.69 (1H, d, *J* = 1.2 Hz), 7.50 (1H, s), 6.86 (1H, s), 6.70 (1H, s), 6.42 (1H, d, *J* = 16.8 Hz), 6.30 (1H, dd, *J* = 16.8 Hz, 10.4 Hz), 5.77 (1H, d, *J* = 10.4 Hz), 3.97 (6H, s), 3.92 (3H, s), 3.71-3.75 (4H, m), 2.90-2.95 (4H, m), 1.74-1.79 (4H, m), 1.61-1.66 (4H, m).

### Example 22

### N-(5-(4-(2,6-dichloro-3,5-dimethoxyphenyl)imidazo[1,2-a][1,6]naphthyridin-8-y l)-4-methoxy-2-(7-methyl-2,7-diazaspiro[4.4]nonan-2-yl)phenyl)acrylamide

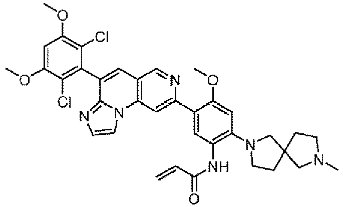

The synthetic method of example 22 was same as that of example 1. ¹H NMR (400 MHz, CDCl₃), 9.08 (1H, s), 8.42 (1H, s), 8.11 (1H, s), 8.09 (1H, s), 7.66 (1H, s), 7.44 (1H, s), 6.67-6.71 (2H, m), 6.49-6.61 (1H, m), 6.41 (1H, d, *J* = 16.8 Hz), 6.36 (1H, s), 5.73 (1H, d, *J* = 10.8 Hz), 3.93-4.01 (13H, m), 3.37-3.56 (4H, m), 2.81 (3H, s), 1.99-2.26 (4H, m).

### Example 23

### N-(5-(4-(2,6-dichloro-3,5-dimethoxyphenyl)imidazo[1,2-a][1,6]naphthyridin-8-y l)-4-methoxy-2-(4-methyl-4,7-diazaspiro[2.5]octan-7-yl)phenyl)acrylamide

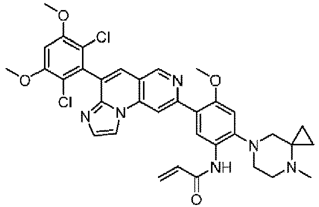

The synthetic method of example 23 was same as that of example 1. ¹H NMR (400 MHz, CDCl₃), 9.21 (1H, s), 9.02 (1H, s), 8.44 (1H, s), 8.36 (1H, s), 8.12 (1H, d, *J* = 1.2 Hz), 7.69 (1H, d, *J* = 1.2 Hz), 7.50 (1H, s), 6.90 (1H, s), 6.70 (1H, s), 6.44 (1H, d, *J* = 16.8 Hz), 6.30 (1H, dd, *J* = 16.8 Hz, 10.0 Hz), 5.78 (1H, d, *J* = 10.0 Hz), 3.98 (6H, s), 3.93 (3H, s), 3.09-3.13 (2H, m), 3.02-3.08 (2H, m), 2.67-2.78 (2H, m), 2.46 (3H, s), 0.84-0.90 (2H, m), 0.49-0.52 (2H, m).

### Example 24

### N-(2-(5-cyclopropylhexahydropyrrolo[3,4-c]pyrrol-2(1H)-yl)-5-(4-(2,6-dichloro-3,5-dimethoxyphenyl)imidazo[1,2-a][1,6]naphthyridin-8-yl)-4-methoxyphenyl)acryla mide

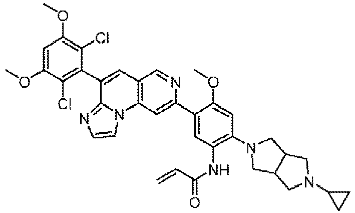

The synthetic method of example 24 was same as that of example 1. ¹H NMR (400 MHz, DMSO), 9.25 (1H, s), 9.05 (1H, s), 8.70 (2H, s), 8.17 (1H, s), 7.69 (1H, s), 7.59 (1H, s), 7.06 (1H, s), 6.61-6.74 (2H, m), 6.21-6.30 (1H, m), 5.69-5.78 (1H, m), 3.94-4.01 (9H, m), 3.54-3.63 (4H, m), 3.06-3.14 (4H, m), 2.90-3.02 (3H, m), 1.20-1.30 (2H, m), 0.74-0.84 (2H, m).

### Example 25

### N-(2-(8-cyclopropyl-2,8-diazaspiro[4.5]decan-2-yl)-5-(4-(2,6-dichloro-3,5-dimet hoxyphenyl)imidazo[1,2-a][1,6]naphthyridin-8-yl)-4-methoxyphenyl)acrylamide

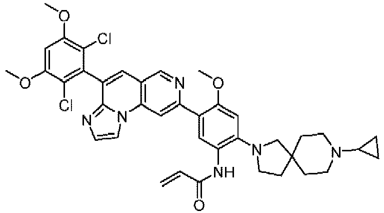

The synthetic method of example 25 was same as that of example 1. ¹H NMR (400 MHz, DMSO), 9.52 (1H, s), 9.20 (1H, s), 8.68 (1H, s), 8.66 (1H, s), 7.91 (1H, s), 7.66 (1H, s), 7.58 (1H, s), 7.06 (1H, s), 6.38-6.51 (2H, m), 6.19 (1H, d, *J* = 16.8 Hz), 5.70 (1H, d, *J* = 10.4 Hz), 3.99 (3H, s), 3.98 (6H, s), 3.38-3.54 (4H, m), 3.14-3.30 (4H, m), 2.76-2.94 (1H, m), 1.69-1.91 (4H, m), 1.39-1.62 (2H, m), 0.96-1.12 (2H, m), 0.69-0.87 (2H, m).

### Example 26

### N-(5-(4-(2,6-dichloro-3,5-dimethoxyphenyl)imidazo[1,2-a][1,6]naphthyridin-8-y l)-4-methoxy-2-(2-oxa-7-azaspiro[4.4]nonan-7-yl)phenyl)but-2-ynamide

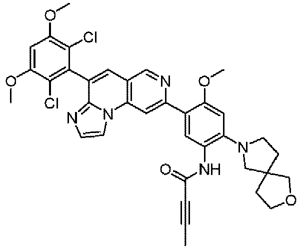

The synthetic method of example 26 was same as that of example 1. ¹H NMR (400 MHz, CDCl₃), 9.15 (1H, s), 8.40 (1H, s), 8.15 (1H, s), 8.11 (1H, s), 7.68 (1H, s), 7.62 (1H, s), 7.46 (1H, s), 6.70 (1H, s), 6.42 (1H, s), 3.89-3.97 (11H, m), 3.75 (1H, d, *J* = 8.4 Hz), 3.67 (1H, d, *J* = 8.4 Hz), 3.44-3.49 (2H, m), 3.34 (1H, d, *J* = 9.2 Hz), 3.30 (1H, d, *J* = 9.2 Hz), 1.88-2.03 (7H, m).

### Example 27

### N-(2-((1S,4S)-2-oxa-5-azabicyclo[2.2.1]heptan-5-yl)-5-(4-(2,6-dichloro-3,5-dim ethoxyphenyl)imidazo[1,2-a][1,6]naphthyridin-8-yl)-4-methoxyphenyl)but-2-ynamide

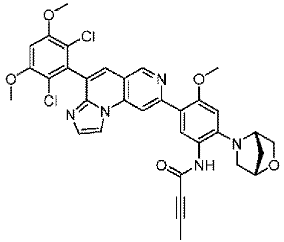

The synthetic method of example 27 was same as that of example 1. ¹H NMR (400 MHz, CDCl₃), 9.16 (1H, s), 8.39 (1H, s), 8.26 (1H, s), 8.11 (1H, s), 7.68 (1H, s), 7.53 (1H, s), 7.47 (1H, s), 6.70 (1H, s), 6.49 (1H, s), 4.65 (1H, s), 4.29 (1H, s), 4.10 (1H, d, *J* = 8.0 Hz), 3.97 (6H, s), 3.93 (3H, s), 3.87 (1H, d, *J* = 8.0 Hz), 3.49 (1H, d, *J* = 9.2 Hz), 3.49 (1H, d, *J* = 9.2 Hz), 1.96-2.06 (5H, m).

### Example 28

### N-(2-(3-oxa-8-azabicyclo[3.2.1]octan-8-yl)-5-(4-(2,6-dichloro-3,5-dimethoxyphe nyl)imidazo[1,2-a][1,6]naphthyridin-8-yl)-4-methoxyphenyl)but-2-ynamide

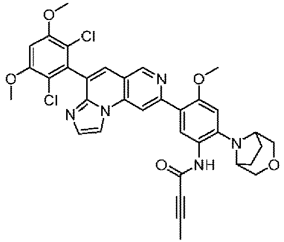

The synthetic method of example 28 was same as that of example 1. ¹H NMR (400 MHz, CDCl₃), 9.19 (1H, s), 8.67 (1H, s), 8.34 (1H, s), 8.11 (1H, s), 7.88 (1H, s), 7.68 (1H, s), 7.49 (1H, s), 6.70 (1H, s), 6.60 (1H, s), 3.93-4.01 (8H, m), 3.89 (3H, s), 3.76-3.79 (2H, m), 3.64-3.68 (2H, m), 2.01-2.17 (7H, m).

### Example 29

### N-(5-(4-(2,6-dichloro-3,5-dimethoxyphenyl)imidazo[1,2-a][1,6]naphthyridin-8-y l)-4-methoxy-2-morpholinophenyl)but-2-ynamide

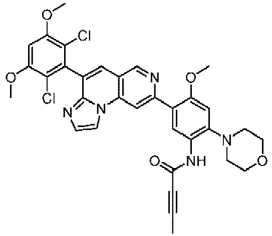

The synthetic method of example 29 was same as that of example 1. ¹H NMR (400 MHz, CDCl₃), 9.21 (1H, s), 8.80 (1H, s), 8.33 (1H, s), 8.25 (1H, s), 8.12 (1H, s), 7.68 (1H, s), 7.49 (1H, s), 6.84 (1H, s), 6.70 (1H, s), 3.96 (6H, s), 3.89-3.95 (7H, m), 2.95-2.98 (4H, m), 2.03 (3H, s).

### Example 30

### N-(5-(4-(2,6-dichloro-3,5-dimethoxyphenyl)imidazo[1,2-a][1,6]naphthyridin-8-y l)-2-(1,1-dioxidothiomorpholino)-4-methoxyphenyl)but-2-ynamide

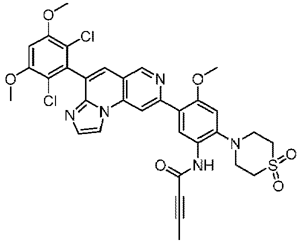

The synthetic method of example 30 was same as that of example 1. ¹H NMR (400 MHz, CDCl₃), 9.22 (1H, s), 8.74 (1H, s), 8.33 (1H, s), 8.12 (1H, s), 7.96 (1H, s), 7.70 (1H, s), 7.51 (1H, s), 6.87 (1H, s), 6.71 (1H, s), 3.98 (6H, s), 3.92 (3H, s), 3.49-3.53 (4H, m), 3.31-3.35 (4H, m), 2.05 (3H, s).

### Example 31

### N-(5-(4-(2,6-dichloro-3,5-dimethoxyphenyl)imidazo[1,2-a][1,6]naphthyridin-8-y l)-4-methoxy-2-(4-morpholinopiperidin-1-yl)phenyl)but-2-ynamide

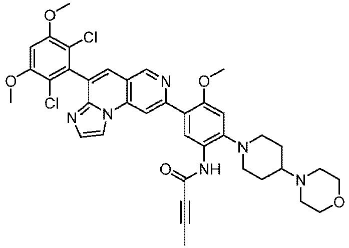

The synthetic method of example 31 was same as that of example 1. ¹H NMR (400 MHz, CDCl₃), 9.21 (1H, s), 8.79 (1H, s), 8.32 (1H, s), 8.17 (1H, s), 8.11 (1H, d, *J* = 1.2 Hz), 7.69 (1H, d, *J* = 1.2 Hz), 7.49 (1H, s), 6.82 (1H, s), 6.70 (1H, s), 3.97 (6H, s), 3.79-3.92 (7H, m), 3.18-3.24 (2H, m), 2.61-2.84 (6H, m), 2.13-2.23 (1H, m), 2.04 (3H, s), 1.59-1.91 (4H, m).

### Example 32

### N-(5-(4-(2,6-dichloro-3,5-dimethoxyphenyl)imidazo[1,2-a][1,6]naphthyridin-8-y l)-4-methoxy-2-(4-methyl-4,7-diazaspiro[2.5]octan-7-yl)phenyl)but-2-ynamide

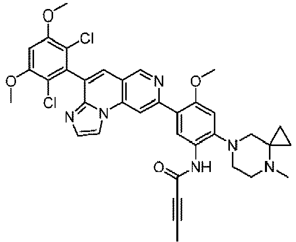

The synthetic method of example 32 was same as that of example 1. ¹H NMR (400 MHz, CDCl₃), 9.21 (1H, s), 8.79 (1H, s), 8.32 (2H, s), 8.11 (1H, d, *J* = 1.2 Hz), 7.69 (1H, d, *J* = 1.2 Hz), 7.49 (1H, s), 6.86 (1H, s), 6.70 (1H, s), 3.98 (6H, s), 3.92 (3H, s), 3.02-3.28 (4H, m), 2.38-2.87 (5H, m), 2.04 (3H, s), 0.79-0.96 (2H, m), 0.61-0.77 (2H, m).

### Reference Example 33

### N-(5-(6-(2,6-dichloro-3,5-dimethoxyphenyl)imidazo[1',2':1,6]pyrido[2,3-d]pyri midin-2-yl)-4-methoxy-2-(4-(oxetan-3-yl)piperazin-1-yl)phenyl)acrylamide

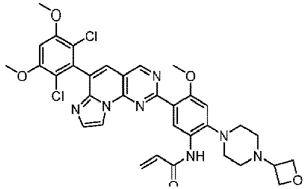

The synthetic method of example 33 was same as that of example 1. ¹H NMR (400 MHz, CDCl₃), 9.34 (1H, s), 9.07 (1H, s), 8.58 (1H, s), 8.26 (1H, s), 7.68(1H, s), 7.43(1H, s), 6.94 (1H, s), 6.71 (1H, s), 6.41 (1H, d, *J* = 16.8 Hz), 6.28 (1H, dd, *J* = 16.8 Hz, 10.4 Hz), 5.77 (1H, d, *J* = 10.4 Hz), 4.66-4.75 (4H, m), 3.98 (6H, s), 3.93 (3H, s), 3.60-3.66 (1H, m), 3.02-3.09 (4H, m), 2.48-2.64 (4H, m).

### Example 34

### N-(5-(4-(2,6-dichloro-3,5-dimethoxyphenyl)imidazo[1,2-a][1,6]naphthyridin-8-y l)-4-methoxy-2-(5-methyl-2,5-diazabicyclo[2.2.1]heptan-2-yl)phenyl)acrylamide

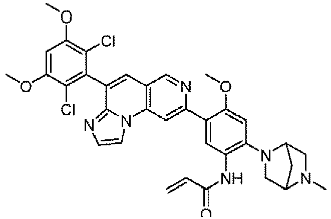

The synthetic method of example 34 was same as that of example 1. ¹H NMR (400 MHz, CDCl₃), 9.07 (1H, s), 8.84 (1H, s), 8.40 (1H, s), 8.35 (1H, s), 8.10 (1H, s), 7.63 (1H, s), 7.42 (1H, s), 6.61-6.75 (3H, m), 6.39 (1H, dd, *J* = 16.8 Hz, 1.2 Hz), 5.69 (1H, d, *J* = 10.4 Hz), 4.44-4.50 (1H, m), 4.10-4.20 (2H, m), 3.91-3.98 (9H, m), 3.64-3.81 (1H, m), 3.43-3.52 (1H, m), 3.19-3.27 (1H, m), 2.87 (3H, s), 2.26-2.38 (2H, m).

### Reference Example 35

### N-(2-(4-cyclopropylpiperazin-1-yl)-5-(6-(2,6-dichloro-3,5-dimethoxyphenyl)imi dazo[1',2':1,6]pyrido[2,3-d]pyrimidin-2-yl)-4-methoxyphenyl)acrylamide

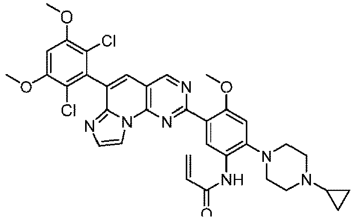

The synthetic method of example 35 was same as that of example 1. ¹H NMR (400 MHz, DMSO), 9.55 (1H, s), 9.17 (1H, s), 8.51 (1H, d, *J* = 1.6 Hz), 8.33 (1H, s), 7.75 (1H, s), 7.64 (1H, d, *J* = 1.6 Hz), 7.09 (1H, s), 6.87 (1H, s), 6.63 (1H, dd, *J* = 16.8 Hz, 10.4 Hz), 6.23 (1H, dd, *J* = 16.8 Hz, 1.6 Hz), 5.73 (1H, dd, *J* = 10.4 Hz, 1.6 Hz), 3.99 (6H, s), 3.89 (3H, s), 2.92-2.97 (4H, m), 2.74-2.79 (4H, m), 1.68-1.74 (1H, m), 0.42-0.47 (2H, m), 0.30-0.36 (2H, m).

### Reference Example 36

### N-(2-((1S,4S)-2-oxa-5-azabicyclo[2.2.1]heptan-5-yl)-5-(6-(2,6-dichloro-3,5-dim ethoxyphenyl)imidazo[1',2':1,6]pyrido[2,3-d]pyrimidin-2-yl)-4-methoxyphenyl)acryl amide

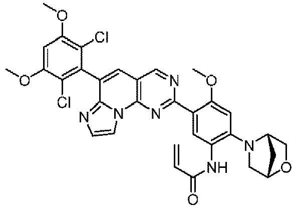

The synthetic method of example 36 was same as that of example 1. ¹H NMR (400 MHz, CDCl₃), 9.31 (1H, s), 8.58 (1H, s), 8.55 (1H, s), 7.69 (1H, s), 7.43 (1H, s), 7.39 (1H, s), 6.72 (1H, s), 6.62 (1H, s), 6.46 (1H, d, *J* = 16.8 Hz), 6.33 (1H, dd, *J* = 16.8 Hz, 10.0 Hz), 5.81 (1H, d, *J* = 10.0 Hz), 4.65-4.69 (1H, m), 4.27-4.31 (1H, m), 4.12 (1H, d, *J* = 8.0 Hz), 3.99 (6H, s), 3.97 (3H, s), 3.88 (1H, d, *J* = 8.0 Hz), 3.42-3.46 (2H, m), 1.99-2.09 (2H, m).

### Example 37

### N-(2-(4-acetylpiperazin-1-yl)-5-(4-(2,6-dichloro-3,5-dimethoxyphenyl)imidazo[ 1,2-a][1,6]naphthyridin-8-yl)-4-methoxyphenyl)acrylamide

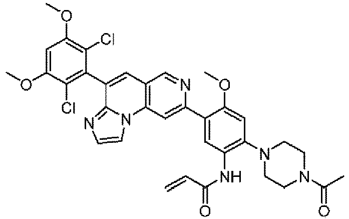

The synthetic method of example 37 was same as that of example 1. ¹H NMR (400 MHz, CDCl₃), 9.21 (1H, s), 8.99 (1H, s), 8.39 (1H, s), 8.24 (1H, s), 8.14 (1H, s), 7.70 (1H, s), 7.51 (1H, s), 6.81 (1H, s), 6.71 (1H, s), 6.43 (1H, d, *J* = 16.8 Hz), 6.30 (1H, dd, *J* = 16.8 Hz, 10.0 Hz), 5.77 (1H, d, *J* = 10.0 Hz), 3.97 (6H, s), 3.94 (3H, s), 3.79-3.87 (2H, m), 3.64-3.70 (2H, m), 2.94-2.99 (4H, m), 2.17 (3H, s).

### Example 38

### (S)-N-(5-(4-(2,6-dichloro-3,5-dimethoxyphenyl)imidazo[1,2-a][1,6]naphthyridin -8-yl)-4-methoxy-2-(2-methylmorpholino)phenyl)acrylamide

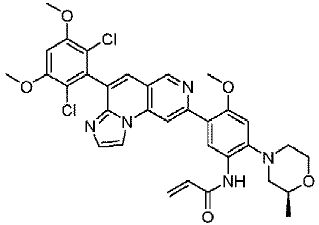

The synthetic method of example 38 was same as that of example 1. ¹H NMR (400 MHz, CDCl₃), 9.21 (1H, s), 9.01 (1H, s), 8.39 (1H, s), 8.28 (1H, s), 8.14 (1H, d, *J* = *1.2* Hz), 7.68 (1H, d, *J* = 1.2 Hz), 7.50 (1H, s), 6.83 (1H, s), 6.70 (1H, s), 6.41 (1H, d, *J* = 16.8 Hz), 6.29 (1H, dd, *J* = 16.8 Hz, 10.0 Hz), 5.76 (1H, dd, *J* = 10.0 Hz, 1.2 Hz), 4.04 (1H, d, *J* = 11.6 Hz), 3.96 (6H, s), 3.92 (3H, s), 3.77-3.86 (2H, m), 2.88-2.95 (3H, m), 2.63 (1H, t, *J* = 10.8 Hz), 1.24 (3H, t, *J* = 6.4 Hz).

### Example 39

### N-(5-(4-(2,6-dichloro-3,5-dimethoxyphenyl)imidazo[1,2-a][1,6]naphthyridin-8-y l)-4-methoxy-2-(4-(methylsulfonyl)piperazin-1-yl)phenyl)acrylamide

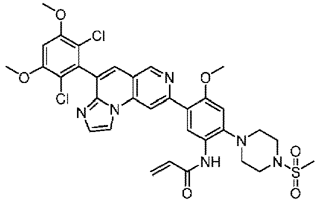

The synthetic method of example 39 was same as that of example 1. ¹H NMR (400 MHz, CDCl₃), 9.19 (1H, s), 8.96 (1H, s), 8.37 (1H, s), 8.11 (1H, d, *J* = 1.6 Hz), 8.10 (1H, s), 7.68 (1H, d, *J* = 1.2 Hz), 7.49 (1H, s), 6.83 (1H, s), 6.69 (1H, s), 6.42 (1H, d, *J* = 16.8 Hz), 6.27 (1H, dd, *J* = 16.8 Hz, 10.0 Hz), 5.76 (1H, d, *J* = 10.0 Hz), 3.96 (6H, s), 3.91 (3H, s), 3.42-3.46 (4H, m), 3.05-3.08 (4H, m), 2.88 (3H, s).

### Example 40

### N-(5-(4-(2,6-dichloro-3,5-dimethoxyphenyl)imidazo[1,2-a][1,6]naphthyridin-8-y l)-2-(2,2-dimethylmorpholino)-4-methoxyphenyl)acrylamide

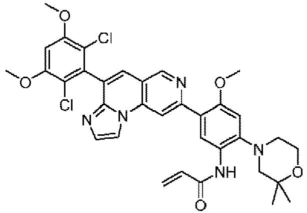

The synthetic method of example 40 was same as that of example 1. ¹H NMR (400 MHz, CDCl₃), 9.22 (1H, s), 8.99 (1H, s), 8.39 (1H, s), 8.22 (1H, s), 8.13 (1H, d, *J* = 1.2 Hz), 7.70 (1H, d, *J* = 1.2 Hz), 7.51 (1H, s), 6.84 (1H, s), 6.71 (1H, s), 6.46 (1H, d, *J* = 16.8 Hz), 6.26 (1H, dd, *J* = 16.8 Hz, 10.4 Hz), 5.78 (1H, d, *J* = 10.4 Hz), 3.92-4.02 (11H, m), 2.97 (2H, t, *J* = 4.4 Hz), 2.71 (2H, s), 1.41 (6H, s).

### Example 41

### (R)-N-(5-(4-(2,6-dichloro-3,5-dimethoxyphenyl)imidazo[1,2-a][1,6]naphthyridin -8-yl)-4-methoxy-2-(2-methylmorpholino)phenyl)acrylamide

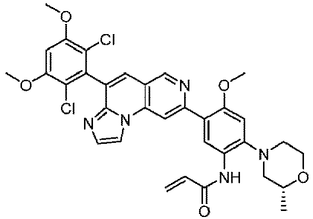

The synthetic method of example 41 was same as that of example 1. ¹H NMR (400 MHz, CDCl₃), 9.21 (1H, s), 8.99 (1H, s), 8.39 (1H, s), 8.27 (1H, s), 8.14 (1H, d, *J* = 1.2 Hz), 7.69 (1H, d, *J* = 1.2 Hz), 7.51 (1H, s), 6.84 (1H, s), 6.70 (1H, s), 6.41 (1H, d, *J* =16.8 Hz), 6.29 (1H, dd, *J* = 16.8 Hz, 10.0 Hz), 5.77 (1H, d, *J* = 10.0 Hz), 4.04 (1H, d, *J* = 11.2 Hz), 3.96 (6H, s), 3.92 (3H, s), 3.78-3.87 (2H, m), 2.88-2.98 (3H, m), 2.63 (1H, dd, *J* = 11.2 Hz, 10.0 Hz), 1.25 (3H, d, *J* = 6.4 Hz).

### Example 42

### N-(2-(4-cyclopropylpiperazin-1-yl)-5-(4-(2,6-dichloro-3,5-dimethoxyphenyl)-2-methylimidazo[1,2-a][1,6]naphthyridin-8-yl)-4-methoxyphenyl)acrylamide

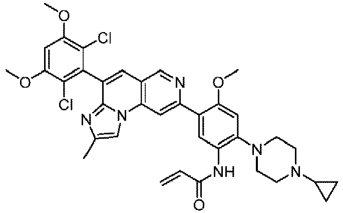

The synthetic method of example 42 was same as that of example 1. ¹H NMR (400 MHz, CDCl₃), 9.17 (1H, s), 8.97 (1H, s), 8.36 (1H, s), 8.28 (1H, s), 7.86 (1H, s), 7.43 (1H, s), 6.85 (1H, s), 6.68 (1H, s), 6.42 (1H, d, *J* = 16.8 Hz), 6.32 (1H, dd, *J* = 16.8 Hz, 10.0 Hz), 5.77 (1H, d, *J* = 10.0 Hz), 3.96 (6H, s), 3.82 (3H, s), 2.75-3.05 (8H, m), 2.47 (3H, s), 1.70-1.81 (1H, m), 0.46-0.61 (4H, m).

### Example 43

### tert-butyl

### 2-(2-acrylamide-4-(4-(2,6-dichloro-3,5-dimethoxyphenyl)imidazo[1,2-a][1,6]naphthy ridin-8-yl)-5-methoxyphenyl)-2,7-diazaspiro[3.5]nonan-7-carboxylate

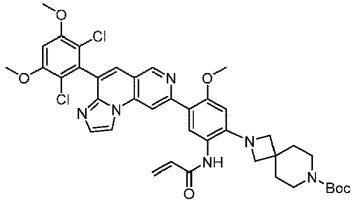

The synthetic method of example 43 was same as that of example 1. ¹H NMR (400 MHz, CDCl₃), 9.11 (1H, s), 8.42 (1H, s), 8.11 (1H, s), 8.04 (1H, s), 7.68 (1H, s), 7.45 (1H, s), 7.03 (1H, s), 6.70 (1H, s), 6.42 (1H, d, *J* = 16.8 Hz), 6.29 (1H, dd *J* = 16.8 Hz, 10.0 Hz), 6.12 (1H, s), 5.78 (1H, d, *J* = 10.0 Hz), 3.97 (6H, s), 3.95 (3H, s), 3.76 (4H, s), 3.35-3.41 (4H, m), 1.72-1.81 (4H, m), 1.46 (9H, s).

### Example 44

### N-(2-(8-oxa-3-azabicyclo[3.2.1]octan-3-yl)-5-(4-(2,6-dichloro-3,5-dimethoxyphe nyl)imidazo[1,2-a][1,6]naphthyridin-8-yl)-4-methoxyphenyl)acrylamide

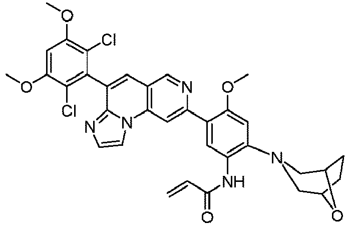

The synthetic method of example 44 was same as that of example 1. ¹H NMR (400 MHz, CDCl₃), 9.22 (1H, s), 9.01 (1H, s), 8.38 (1H, s), 8.36 (1H, s), 8.13 (1H, d, *J* = 1.2 Hz), 7.71 (1H, d, *J* = 1.2 Hz), 7.51 (1H, s), 6.93 (1H, s), 6.72 (1H, s), 6.47 (1H, d, *J* = 16.8 Hz), 6.30 (1H, dd, *J* = 16.8 Hz, 10.0 Hz), 5.81 (1H, d, *J* = 10.0 Hz), 4.52 (2H, s), 3.99 (6H, s), 3.92 (3H, s), 3.19 (2H, d, *J* = 11.2 Hz), 2.78 (2H, d, *J* = 11.2 Hz), 2.09-2.05 (4H, m).

### Example 45

### N-(5-(4-(2,6-dichloro-3,5-dimethoxyphenyl)imidazo[1,2-a][1,6]naphthyridin-8-y l)-4-methoxy-2-(2,7-diazaspiro[3.5]nonan-2-yl)phenyl)acrylamide hydrochloride

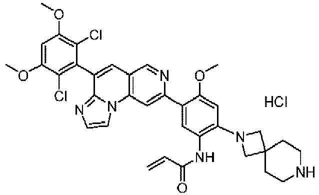

The synthetic method of example 45 was same as that of example 1. ¹H NMR (400 MHz, CD₃OD), 9.49 (1H, s), 9.04 (1H, s), 9.01 (1H, s), 8.22 (1H, s), 8.02 (1H, s), 7.91 (1H, s), 7.08 (1H, s), 6.51 (1H, dd, *J* = 16.8 Hz, 10.0 Hz), 6.41 (1H, dd, *J* = 16.8 Hz, 2.0 Hz), 6.27 (1H, s), 5.86 (1H, dd, *J* = 10.0 Hz, 2.0 Hz), 4.07 (3H, s), 4.04 (6H, s), 3.95 (4H, s), 3.21-3.24 (4H, m), 2.07-2.11 (4H, m).

### Example 46

### N-(2-(((4-(chloromethyl)piperidin-4-yl)methyl)amino)-5-(4-(2,6-dichloro-3,5-di methoxyphenyl)imidazo[1,2-a][1,6]naphthyridin-8-yl)-4-methoxyphenyl)acrylamide hydrochloride

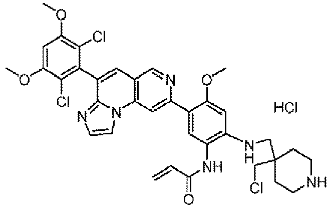

The synthetic method of example 46 was same as that of example 1. ¹H NMR (400 MHz, CD₃OD), 9.59 (1H, s), 9.20 (1H, d, *J* = 2.0 Hz), 9.09 (1H, s), 8.36 (1H, s), 8.14 (1H, d, *J* = 2.0 Hz), 7.97 (1H, s), 7.11 (1H, s), 6.75 (1H, s), 6.62 (1H, dd, *J* = 16.8 Hz, 10.4 Hz), 6.41 (1H, dd, *J* = 16.8 Hz, 1.2 Hz), 5.85 (1H, dd, *J* = 10.4 Hz, 1.2 Hz), 4.13 (3H, s), 4.05 (6H, s), 3.83 (2H, s), 3.53 (2H, s), 3.30-3.36 (2H, m), 3.19-3.25 (2H, m), 1.90-2.03 (4H, m).

### Example 47

### N-(2-(4-cyclopropylpiperazin-1-yl)-5-(4-(2,6-dichloro-3,5-dimethoxyphenyl)imi dazo[1,2-a][1,6]naphthyridin-8-yl)-4-methoxyphenyl)but-2-ynamide

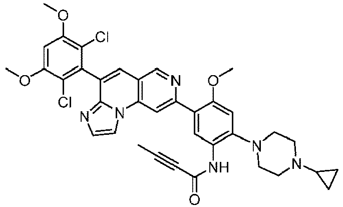

The synthetic method of example 47 was same as that of example 1. ¹H NMR (400 MHz, CDCl₃), 9.22 (1H, s), 8.82 (1H, s), 8.34 (1H, s), 8.30 (1H, s), 8.13 (1H, s), 7.71 (1H, s), 7.51 (1H, s), 6.88 (1H, s), 6.72 (1H, s), 3.99 (6H, S), 3.89 (3H, s), 2.78-3.11 (8H, m), 2.06 (3H, s), 1.77-1.84 (1H, m), 0.44-0.64 (4H, m).

### Example 48

### (Z)-N-(2-(4-cyclopropylpiperazin-1-yl)-5-(4-(2,6-dichloro-3,5-dimethoxyphenyl) imidazo[1,2-a][1,6]naphthyridin-8-yl)-4-methoxyphenyl)-4-methoxybut-2-enamide

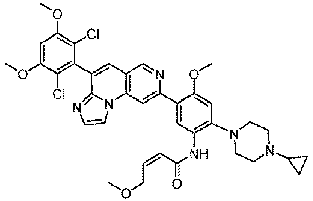

The synthetic method of example 48 was same as that of example 1. ¹H NMR (400 MHz, CDCl₃), 9.21 (1H, s), 8.89 (1H, s), 8.44 (1H, s), 8.33 (1H, s), 8.11 (1H, s), 7.68 (1H, s), 7.49 (1H, s), 6.85 (1H, s), 6.70 (1H, s), 6.25 (1H, d, *J* = 6.0 Hz), 4.66-4.72 (1H, m), 3.98 (6H, s), 3.87 (3H, s), 3.72 (3H, s), 3.24 (2H, d *J* = 7.6 Hz), 2.77-3.24 (8H, m), 1.78-1.87 (1H, m), 0.47-0.59 (4H, m).

### Example 49

### 2-chloro-N-(2-(4-cyclopropylpiperazin-1-yl)-5-(4-(2,6-dichloro-3,5-dimethoxyp henyl)imidazo[1,2-a][1,6]naphthyridin-8-yl)-4-methoxyphenyl)acetamide

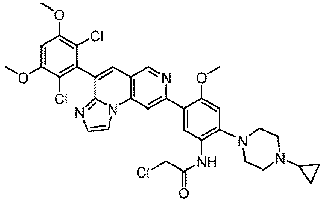

The synthetic method of example 49 was same as that of example 1. ¹H NMR (400 MHz, CDCl₃), 9.48 (1H, s), 9.22 (1H, s), 8.96 (1H, s), 8.37 (1H, s), 8.12 (1H, d, *J* = 1.2 Hz), 7.70 (1H, d, *J* = 1.2 Hz), 7.50 (1H, s), 6.92 (1H, s), 6.71 (1H, s), 4.27 (2H, s), 3.98 (6H, s), 3.84 (3H, s), 2.85-3.08 (8H, m), 1.76-1.82 (1H, m), 0.49-0.63 (4H, m).

### Example 50

### 2-cyano-N-(2-(4-cyclopropylpiperazin-1-yl)-5-(4-(2,6-dichloro-3,5-dimethoxyph enyl)imidazo[1,2-a][1,6]naphthyridin-8-yl)-4-methoxyphenyl)acetamide

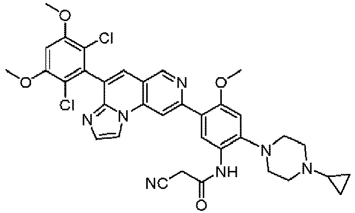

The synthetic method of example 50 was same as that of example 1. ¹H NMR (400 MHz, CDCl₃), 9.28 (1H, s), 9.23 (1H, s), 8.92 (1H, s), 8.37 (1H, s), 8.12 (1H, s), 7.70 (1H, s), 7.51 (1H, s), 6.95 (1H, s), 6.71 (1H, s), 3.98 (6H, s), 3.90(3H, s), 3.62 (2H, s), 2.86-3.17 (8H, m), 1.76-1.95 (1H, m), 0.50-0.79 (4H, m).

### Example 51

### N-(2-(4-cyclopropylpiperazin-1-yl)-5-(4-(2,6-dichloro-3,5-dimethoxyphenyl)imi dazo[1,2-a][1,6]naphthyridin-8-yl)-4-methoxyphenyl)but-3-ynamide

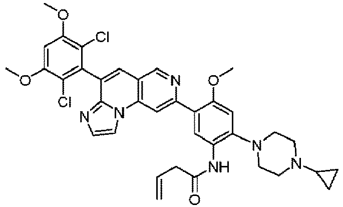

The synthetic method of example 51 was same as that of example 1. ¹H NMR (400 MHz, CDCl₃), 9.21 (1H, s), 8.92 (1H, s), 8.41 (1H, s), 8.34 (1H, s), 8.11 (1H, d, *J* = 1.6 Hz), 7.69 (1H, d *J* = 1.2 Hz), 7.49 (1H, s), 6.87 (1H, s), 6.70 (1H, s), 6.03-6.14 (1H, m), 5.38-5.43 (2H, m), 3.98 (6H, s), 3.87 (3H, s), 3.25 (2H, d, *J* = 7.6 Hz), 2.77-3.02 (8H, m), 1.69-1.89 (1H, m), 0.48-0.61 (4H, m).

### Example 52

### (E)-N-(2-(4-cyclopropylpiperazin-1-yl)-5-(4-(2,6-dichloro-3,5-dimethoxyphenyl) imidazo[1,2-a][1,6]naphthyridin-8-yl)-4-methoxyphenyl)but-2-enamide

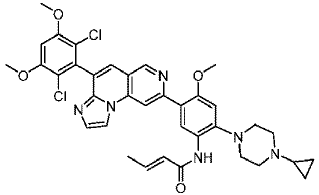

The synthetic method of example 52 was same as that of example 1. ¹H NMR (400 MHz, CDCl₃), 9.33 (1H, s), 9.11 (1H, s), 8.48 (1H, s), 8.23-8.26 (2H, m), 7.81 (1H, d, *J* = 1.2 Hz), 7.62 (1H, s), 7.08-7.17 (1H, m), 7.00 (1H, s), 6.83 (1H, s), 6.12 (1H, d, *J* = 16.8 Hz), 4.10 (6H, s), 4.01 (3H, s), 2.90-3.20 (8H, m), 2.07 (3H, d, *J* = 6.8 Hz), 1.83-1.96 (1H, m), 0.57-0.80 (4H, m).

### Example 53

### N-(2-(4-cyclopropylpiperazin-1-yl)-5-(4-(2,6-dichloro-3,5-dimethoxyphenyl)imi dazo[1,2-a][1,6]naphthyridin-8-yl)-4-methoxyphenyl)propiolamide

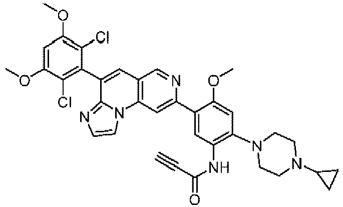

The synthetic method of example 53 was same as that of example 1. ¹H NMR (400 MHz, CDCl₃), 9.23 (1H, s), 8.85 (1H, s), 8.49-8.55 (1H, brs), 8.35 (1H, s), 8.13 (1H, s), 7.71 (1H, s), 7.51 (1H, s), 6.90 (1H, s), 6.72 (1H, s), 3.99 (6H, s), 3.91 (3H, s), 2.85-3.07 (9H, m), 1.74-1.83 (1H, m), 0.46-0.61 (4H, m).

### Bioactivity assay

### 1. In vitro determination of the protein activity of compounds:

The FGFR-4 kinase activity detection platform was established by the homogeneous time-resolved fluorescence (HTRF) method, and the activity of the compound was determined. 1000 µM compounds were subjected to 3-fold gradient dilution in 100% DMSO 11 times (12 concentrations in total), 4 µL diluent from each concentration was added to 96 µL of reaction buffer (50 mM HEPES, pH 7.4, 5 mM MnCl₂, 0.1 mM NaVO₃, 0.001% Tween-20, 0.01% BAS, 1 mM DTT) and mixed well, as a 4* compound (final concentration of 0.017 nM-1000 nM) for later use. 2 * FGFR-4 kinase (final concentration of 1 nM) was formulated using reaction buffer and 4 * substrate (ATP+TK peptide) (TK peptide, HTRF^{®} KinEASE^{™}-TK, purchased from Cisbio, TK peptide, final concentration of 1 µ M, ATP final concentration of 25 µ M) was formulated using reaction buffer. 2.5 µL of 4 * compound and then 5 µL of 2 * FGFR-4 kinase were added to a 384-well plate (OptiPlate-384, purchased from PerkinElmer), the mixture was mixed by centrifugation, and 2.5 µL of 4 * substrate mixture was added to start the reaction (total reaction volume of 10 µL). The 384-well plate was placed in an incubator 23°C and stirred for 60 min before the reaction was stopped by the addition of 5 µL of Eu³⁺ cryptate-labeled anti-phosphotyrosine antibody (TRF^{®} KinEASE^{™}-TK, purchased from Cisbio) and 5 µL of Streptavidin-XL-665 (HTRF^{®} KinEASE^{™}-TK, purchased from Cisbio). After incubation in the incubator for 1 h, fluorescence values (excitation at 320 nm, detecting emitted light at 665 nm and 620 nm, and a ratio of the two as enzyme activity) were read on Envision (purchased from PerkinElmer). The enzyme activity of each compound was measured at 12 concentrations, and the IC₅₀ value of the compound was calculated using GraphPad Prism 5.0 software.

### 2. Determination of cell proliferation activity of compounds:

Cell Titer-Glo ^{®} detection reagent from Promega was used to establish the suspension cell proliferation inhibition screening method. Human hepatoma cells Hep3b (Xiehe Cell Research Center) were cultured in 25 cm² or 75 cm² plastic tissue culture flasks (Corning^{®}) filled with MEM (Gibco^{®}) medium supplemented with 10% fetal bovine serum (Hyclone^{®}) under 37°C, 95% air and 5% CO₂, and subcultured 2-3 times a week.

Hep3b cells were seeded in a 96-well cell culture plate (Corning^{®}) at a density of 3×10³ cells/well, 195 mL/well, and cultured under 37°C, 95% air, and 5% CO₂. After 24 h the test compound was added: 10 mM compounds (dissolved in DMSO) were subjected to 3-fold gradient dilution in DMSO , 4 mL of diluent from each concentration was added to 96 mL of serum-free medium, and finally 5 mL of compound diluted in the medium was added to the plate seeded with cells. The final concentration of DMSO in the cell culture medium was 0.1% and the final concentration of the test compound was 0.3 nM-10 mM. The above cells were incubated for 3 days under 37°C.

After 3 days, the cell viability assay was performed by the Cell Titer-Glo (Promega) kit, and finally, the inhibitory concentration 50% of the compound on cell proliferation, i.e. the IC 50 value, was calculated by the GraphPad Prism 5.0 program.

**Table 1. Inhibitory effects of the compounds in Examples on FGFR4**

| Compound | FGFR4 IC₅₀ (nM) | Hep3B IC₅₀ (nM) |
|---|---|---|
| Example 4 | 2.25 | - |
| Example 5 | - | 20.3 |
| Example 6 | - | 58.3 |
| Example 7 | 1.74 | 83.6 |
| Example 9 | 2.43 | 74.7 |
| Example 11 | 3.82 | 68.3 |
| Example 19 | 5.79 | 71.7 |
| Example 20 | 2.54 | - |
| Example 22 | 5.61 | - |
| Example 23 | 3.85 | 142 |
| Example 29 | 2.17 | 34.4 |
| Example 30 | 0.947 | 46.3 |
| Example 31 | 3.63 | 81.3 |
| Example 32 | 6.10 | 76.8 |
| Example 37 | 2.16 | 107 |
| Example 38 | 5.07 | - |
| Example 44 | 5.03 | 113 |
| Blu-554 | 7.49 | 162 |

### 3. Pharmacokinetic data

Male SD rats (Beijing Vital River Laboratory Animal Technology Co., Ltd.) were divided into groups of 3 rats each. The rats were subjected to 5 mg/kg suspension of the test sample by single intragastric administration, respectively. Animals were fasted overnight prior to the experiment from 10 h before dosing to 4 h after dosing. Blood was collected 0.25, 0.5, 1, 2, 4, 6, 8, and 24 h after administration. After isoflurane anesthesia using a small animal anesthesia machine, 0.3 mL of whole blood samples was collected from the fundus venous plexus, placed in a heparin anticoagulant tube, and were centrifuged at 4°C, 4000 rpm for 5 min, plasma was transferred to the centrifuge tube, and stored at -80°C until analysis. Samples in the plasma were extracted using protein precipitation and the extracts were analyzed by LC/MS/MS.

| Example | Example 6 | Example 27 | Blu-554 |
|---|---|---|---|
| Dose (mg/kg) | 5 | 5 | 5 |
| T_{1/2} (hr) | 11.5 | 2.73 | 1.64 |
| Tmax (hr) | 6.00 | 3.33 | 2.67 |
| Cmax (ng/mL) | 236 | 452 | 362 |
| AUC0-inf (hr*ng/mL) | 2555 | 1858 | 1683 |

### Example 54

### N-(5-(4-(2,6-dichloro-3,5-dimethoxyphenyl)imidazo[1,2-a][1,6]naphthyridin-8-yl)-4-methoxy-2-morpholinophenyl)acrylamide

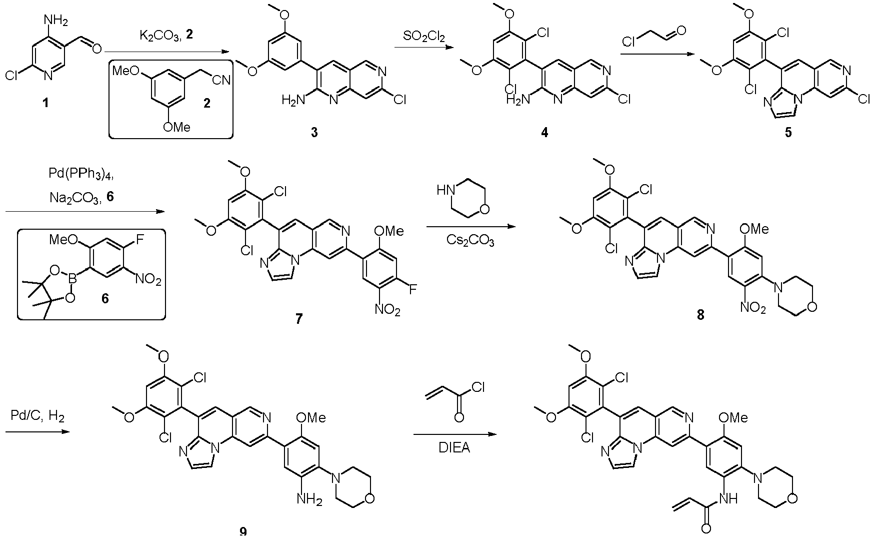

### Step 1: synthesis of compound 3

To a solution of compound **1** (2 g) in 1-Methyl-2-pyrrolidinone (15 mL) were added compound **2** (2.49 g) and potassium carbonate (5.29 g). The reaction solution was heated at 100 °C overnight. The reaction solution was cooled to room temperature and poured into water. The mixture was filtered, and the filter cake was washed with distilled water and dried to obtain compound **3** (3.58 g).

### Step 2: synthesis of compound 4

At -20 °C, to a solution of compound **3** (3.58 g) in THF (100 mL) was added sulfuryl chloride (2.3 mL). The reaction solution was stirred at -20 °C for 2 hours until the reaction was completed. An aqueous solution of saturated sodium bicarbonate was used to quench the reaction. The organic phase was separated, and the aqueous phase was extracted with ethyl acetate. The organic phases were combined, dried over anhydrous sodium sulfate, and concentrated under reduced pressure. The residue thus obtained was purified through flash column chromatography (dichloromethane/methanol = 100/1) to obtain compound **4** (3.24 g).

### Step 3: synthesis of compound 5

To a solution of compound **4** (3.24 g) in ethanol (18 mL) was added chloroacetaldehyde (18 mL). The reaction solution was heated to 80 °C and reacted overnight. The reaction solution was poured into water and filtered. The solid thus obtained was dried to obtain compound **5** (3.1 g).

### Step 4: synthesis of compound 7

Under nitrogen protection, to a solution of compound **5** (1.23 g) in dioxane/water (20 mL/5 mL) were added tetrakis(triphenylphosphine)palladium (500 mg), compound **6** (1.5 g) and anhydrous sodium carbonate (1.5 g). The reaction solution was heated to 80 °C and reacted overnight. The reaction solution was cooled to room temperature and concentrated under reduced pressure, the residue thus obtained was separated and purified through flash silica gel column chromatography (dichloromethane/methanol = 500/1) to obtain compound **7** (1.12 g).

### Step 5: synthesis of compound 8

To a solution of compound **7** (200 mg) in DMF (1 mL) were added anhydrous caesium carbonate (120 mg) and morpholine (0.1 mL), and the mixture was heated to 60 °C and reacted overnight. The reaction solution was cooled to room temperature and concentrated under reduced pressure. The residue thus obtained was separated and purified through flash silica gel column chromatography (dichloromethane/methanol = 100/1 to 50/1) to obtain compound **8** (120 mg).

### Step 6: synthesis of compound 9

To a solution of compound **8** (120 mg) in tetrahydrofuran (5 mL) was added palladium on carbon (50 mg), and the system was purged with hydrogen gas. The reaction system was reacted at room temperature overnight and filtered through celite. The filtrate was concentrated under reduced pressure to obtain compound **9** (71 mg).

### Step 7: synthesis of example 54

At 0 °C, to a solution of compound **9** (71 mg) in dichloromethane (2 mL) were added DIEA (5 µL) and acryloyl chloride (11 µL). The reaction solution was stirred at 0 °C for 2 hours, methanol was added to quench the reaction, and the mixture was concentrated under reduced pressure. The residue thus obtained was separated and purified through preparative thin layer chromatography (dichloromethane/methanol = 25/1) to obtain example **54** (35 mg). ¹H NMR (400 MHz, CDCl₃) δ 9.22 (1H, s), 9.02 (1H, s), 8.38 (1H, s), 8.27 (1H, s), 8.14 (1H, s), 7.70 (1H, s), 7.51 (1H, s), 6.86 (1H, s), 6.71 (1H, s), 6.42 (1H, d, *J* = 16.8 Hz), 6.30 (1H, dd, *J* = 16.8, 10.0 Hz), 5.77 (1H, d, *J* = 10.0 Hz), 3.97 (6H, s), 3.87-3.95 (7H, m), 2.95-3.02 (4H, m).

### Example 55

### N-(5-(4-(2,6-dichloro-3,5-dimethoxyphenyl)imidazo[1,2-a][1,6]naphthyridin-8-yl)-4-methoxy-2-((4-methylpiperazin-1-yl)methyl)phenyl)acrylamide

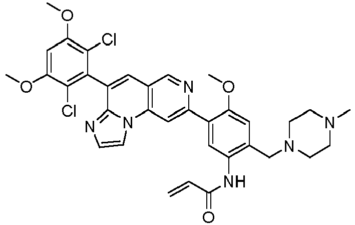

The synthetic method of example 55 was same as that of example 54. ¹H NMR (400 MHz, CDCl₃) δ 9.62-10.21 (1H, brs), 9.20 (1H, s), 8.82 (1H, s), 8.39 (1H, s), 8.15 (1H, s), 7.70 (1H, s), 7.51 (1H, s), 6.88 (1H, s), 6.71 (1H, s), 6.39 (1H, d, *J* = 16.8 Hz), 6.18-6.29 (1H, m), 5.75 (1H, d, *J* = 11.6 Hz), 3.98 (6H, s), 3.92 (3H, s), 3.78 (2H, s), 2.75-3.02 (8H, m), 2.69 (3H, s).

### Example 56

### N-(5-(4-(2,6-dichloro-3,5-dimethoxyphenyl)imidazo[1,2-a][1,6]naphthyridin-8-yl)-2-(3-hydroxy-8-azabicyclo[3.2.1]octan-8-yl)-4-methoxyphenyl)acrylamide

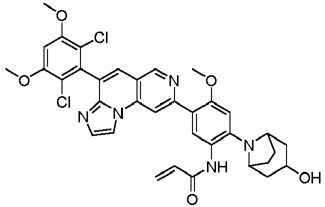

The synthetic method of example 56 was same as that of example 54. ¹H NMR (400 MHz, CDCl₃) δ 9.19 (1H, s), 8.87 (1H, s), 8.38 (1H, s), 8.12 (1H, s), 7.75 (1H, s), 7.69 (1H, s), 7.49 (1H, s), 6.71 (1H, s), 6.66 (1H, s), 6.45 (1H, d, *J* = 16.8 Hz), 6.29 (1H, dd, *J* = 16.8, 10.4 Hz), 5.77 (1H, d, *J* = 10.4 Hz), 4.24-4.29 (1H, m), 3.98 (6H, s), 3.91 (3H, s), 3.75-3.83 (2H, m), 2.32-2.39 (2H, m), 2.21-2.31 (2H, m), 2.04-2.12 (2H, m), 1.94-2.03 (2H, m).

### Example 57

### N-(2-(4-(cyclopropylmethyl)piperazin-1-yl)-5-(4-(2,6-dichloro-3,5-dimethoxyphenyl)imidazo[1,2-a][1,6]naphthyridin-8-yl)-4-methoxyphenyl)acrylamide

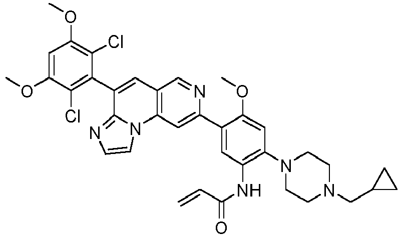

The synthetic method of example 57 was same as that of example 54. ¹H NMR (400 MHz, CDCl₃) δ 9.21 (1H, s), 9.04 (1H, s), 8.37 (1H, s), 8.33 (1H, s), 8.12 (1H, s), 7.70 (1H, s), 7.50 (1H, s), 6.93 (1H, s), 6.71 (1H, s), 6.42 (1H, d, *J* = 17.2 Hz), 6.29 (1H, dd, *J* = 17.2, 10.0 Hz), 5.77 (1H, d, *J* = 10.0 Hz), 3.98 (6H, s), 3.91 (3H, s), 3.01-3.08 (4H, m), 2.67-2.85 (4H, m), 2.39 (2H, d, *J* = 6.4 Hz), 0.81-0.94 (1H, m), 0.55-0.63 (2H, m), 0.15-0.23 (2H, m).

### Example 58

### N-(5-(4-(2,6-dichloro-3,5-dimethoxyphenyl)imidazo[1,2-a][1,6]naphthyridin-8-yl)-4-methoxy-2-(4-morpholinopiperidin-1-yl)phenyl)acrylamide

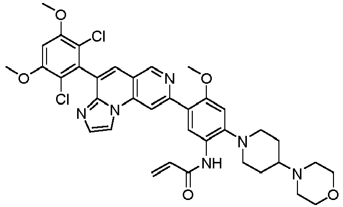

The synthetic method of example 58 was same as that of example 54. ¹H NMR (400 MHz, CDCl₃) δ 9.21 (1H, s), 9.01 (1H, s), 8.36 (1H, s), 8.19 (1H, s), 8.12 (1H, s), 7.69 (1H, s), 7.50 (1H, s), 6.85 (1H, s), 6.71 (1H, s), 6.42 (1H, d, *J* = 16.8 Hz), 6.28 (1H, dd, *J* = 16.8, 10.0 Hz), 5.76 (1H, d, *J* = 10.0 Hz), 3.98 (6H, s), 3.91 (3H, s), 3.76-3.83 (4H, m), 3.20 (2H, d, *J* = 10.8 Hz), 2.79 (2H, t, *J* = 11.6 Hz), 2.61-2.71 (4H, m), 2.30-2.42 (1H, m), 2.12 (2H, d, *J* = 11.6 Hz), 1.64-1.78 (2H, m).

### Example 59

### N-(2-(4-cyclopropylpiperidin-1-yl)-5-(4-(2,6-dichloro-3,5-dimethoxyphenyl)imidazo[1,2-a][1,6]naphthyridin-8-yl)-4-methoxyphenyl)acrylamide

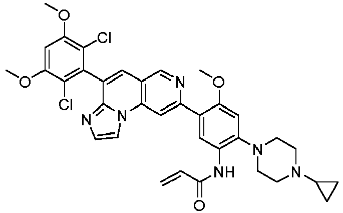

The synthetic method of example 59 was same as that of example 54. ¹H NMR (400 MHz, CDCl₃) δ 9.21 (1H, s), 9.03 (1H, s), 8.30-8.38 (2H, m), 8.12 (1H, s), 7.69 (1H, s), 7.50 (1H, s), 6.89 (1H, s), 6.71 (1H, s), 6.43 (1H, d, *J* = 16.8 Hz), 6.30 (1H, dd, *J* = 16.8 Hz), 5.78 (1H, d, *J* = 10.0 Hz), 3.98 (6H, s), 3.89 (3H, s), 2.94-3.04 (4H, m), 2.79-2.93 (4H, m), 1.72-1.82 (1H, m), 0.42-0.59 (4H, m).

### Example 60

### N-(2-(4-cyanopiperazin-1-yl)-5-(4-(2,6-dichloro-3,5-dimethoxyphenyl)imidazo[1,2-a][1,6]naphthyridin-8-yl)-4-methoxyphenyl)acrylamide

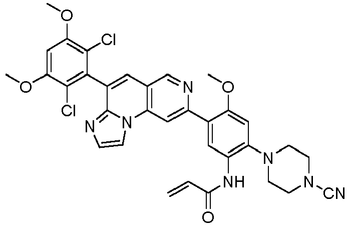

The synthetic method of example 60 was same as that of example 54. ¹H NMR (400 MHz, CDCl₃) δ 9.21 (1H, s), 8.99 (1H, s), 8.37 (1H, s), 8.13 (1H, s), 8.05 (1H, s), 7.70 (1H, s), 7.51 (1H, s), 6.84 (1H, s), 6.71 (1H, s), 6.43 (1H, d, *J* = 17.2 Hz), 6.28 (1H, dd, *J* = 17.2, 10.4 Hz), 5.74 (1H, d, *J* = 10.4 Hz), 3.98 (6H, s), 3.93 (3H, s), 3.43-3.53 (4H, m), 3.03-3.12 (4H, m).

### Example 61

### N-(2-((1S,4S)-2-oxa-5-azabicyclo[2.2.1]heptan-5-yl)-5-(4-(2,6-dichloro-3,5-dimethoxyphenyl)imidazo[1,2-a][1,6]naphthyridin-8-yl)-4-methoxyphenyl)acrylamide

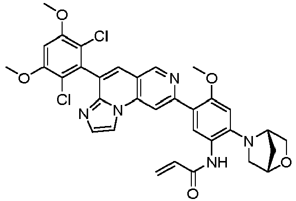

The synthetic method of example 61 was same as that of example 54. ¹H NMR (400 MHz, CDCl₃) δ 9.15 (1H, s), 8.41 (1H, s), 8.40 (1H, s), 8.13 (1H, s), 7.69 (1H, s), 7.60 (1H, s), 7.48 (1H, s), 6.70 (1H, s), 6.52 (1H, s), 6.41 (1H, d, *J* = 16.8 Hz), 6.31 (1H, dd, *J* = 16.8, 10.0 Hz), 5.75 (1H, d, *J* = 10.0 Hz), 4.62 (1H, s), 4.24 (1H, s), 4.08 (1H, d, *J* = 7.6 Hz), 3.97 (6H, s), 3.92 (3H, s), 3.83 (1H, d, *J* = 7.6 Hz), 3.42 (1H, d, *J* = 9.6 Hz), 3.37 (1H, d, *J* = 9.6 Hz), 1.94-2.01 (2H, m).

### Example 62

### N-(5-(4-(2,6-dichloro-3,5-dimethoxyphenyl)imidazo[1,2-a][1,6]naphthyridin-8-yl)-4-methoxy-2-(2-oxa-7-azaspiro[4,4]nonan-7-yl)phenyl)acrylamide

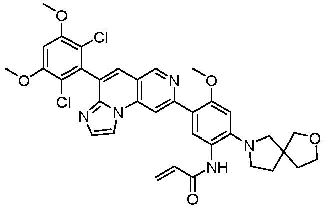

The synthetic method of example 62 was same as that of example 54. ¹H NMR (400 MHz, CDCl₃) δ 9.14 (1H, s), 8.42 (1H, s), 8.28 (1H, s), 8.12 (1H, s), 7.68 (1H, s), 7.63 (1H, s), 7.47 (1H, s), 6.70 (1H, s), 6.46 (1H, s), 6.37 (1H, d, *J* = 16.8 Hz), 6.31 (1H, dd, *J* = 16.8, 10.8 Hz), 5.76 (1H, d, *J* = 10.8 Hz), 3.97 (6H, s), 3.94 (3H, s), 3.87-3.93 (2H, m), 3.72 (1H, d, *J* = 8.4 Hz), 3.65 (1H, d, *J* = 8.4 Hz), 3.37-3.43 (2H, m), 3.30 (1H, d, *J* = 9.2 Hz), 3.25 (1H, d, *J* = 9.2 Hz), 1.86-2.03 (4H, m).

### Example 63

### N-(5-(4-(2,6-dichloro-3,5-dimethoxyphenyl)imidazo[1,2-a][1,6]naphthyridin-8-yl)-2-fluoro-4-methoxyphenylacrylamide

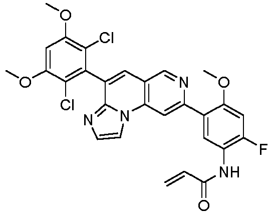

The synthetic method of example 63 was same as that of example 54. ¹H NMR (400 MHz, CDCl₃) δ 9.21 (1H, s), 8.86 (1H, d, *J* = 9.2 Hz), 8.37 (1H, s), 8.14 (1H, s), 7.71 (1H, s), 7.51 (1H, s), 7.46 (1H, s), 6.87 (1H, d, *J* = 12.4 Hz), 6.71 (1H, s), 6.46 (1H, d, *J* = 16.8 Hz), 6.30 (1H, dd, *J* = 16.8, 10.0 Hz), 5.80 (1H, d, *J* = 10.0 Hz), 3.98 (6H, s), 3.93 (3H, s).

### Example 64

### N-(5-(4-(2,6-dichloro-3,5-dimethoxyphenyl)imidazo[1,2-a][1,6]naphthyridin-8-yl)-4-methoxy-2-(4-(4-methylpiperazin-1-yl)piperidin-1-yl)phenyl)acrylamide

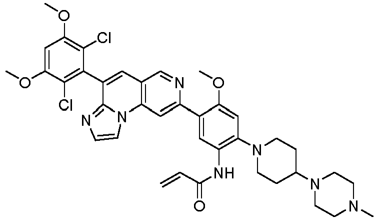

The synthetic method of example 64 was same as that of example 54. ¹H NMR (400 MHz, DMSO-*d₆*) δ 9.51 (1H, s), 9.32 (1H, s), 9.12 (1H, s), 8.93 (1H, s), 8.38 (1H, s), 8.21 (1H, s), 8.08 (1H, s), 7.15 (1H, s), 6.90 (1H, s), 6.89 (1H, dd, *J* = 17.2, 10.0 Hz), 6.25 (1H, d, *J* = 17.2 Hz), 5.75 (1H, d, *J* =10.0 Hz), 4.01 (6H, s), 3.97 (3H, s), 3.68-3.86 (4H, m), 3.49-3.65 (4H, m), 3.32-3.48 (3H, m), 2.75-2.88 (5H, m), 2.14-2.26 (2H, m), 1.96-2.12 (2H, m).

### Example 65

### N-(5-(4-(2,6-dichloro-3,5-dimethoxyphenyl)imidazo[1,2-a][1,6]naphthyridin-8-yl)-4-methoxy-2-(4-(oxetan-3-yl)piperazin-1-yl)phenyl)acrylamide

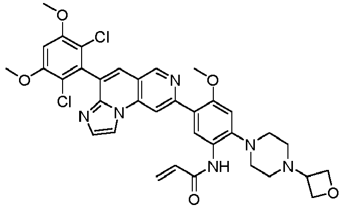

The synthetic method of example 65 was same as that of example 54. ¹H NMR (400 MHz, CDCl₃) δ 9.21 (1H, s), 9.02 (1H, s), 8.37 (1H, s), 8.24 (1H, s), 8.12 (1H, s), 7.70 (1H, s), 7.50 (1H, s), 6.91 (1H, s), 6.71 (1H, s), 6.42 (1H, d, *J* = 16.8 Hz), 6.28 (1H, dd, *J* = 16.8, 10.0 Hz), 5.77 (1H, d, *J* = 10.0 Hz), 4.67-4.75 (4H, m), 3.98 (6H, s), 3.92 (3H, s), 3.60-3.68 (1H, m), 3.01-3.12 (4H, m), 2.46-2.67 (4H, m).

### Example 66

### N-(5-(4-(2,6-dichloro-3,5-dimethoxyphenyl)imidazo[1,2-a][1,6]naphthyridin-8-yl)-2-((2-(dimethylamino)ethyl)(methyl)amino)-4-methoxyphenyl)acrylamide

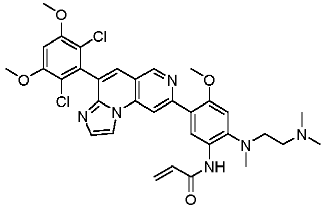

The synthetic method of example 66 was same as that of example 54. ¹H NMR (400 MHz, CD₃OD) δ 9.20 (1H, s), 8.54 (1H, s), 8.35 (1H, s), 8.24 (1H, s), 7.65 (1H, s), 7.56 (1H, s), 6.93 (1H, s), 6.85 (1H, s), 6.69 (1H, dd, *J* = 17.2, 10.4 Hz), 6.43 (1H, d, *J* = 17.2 Hz), 5.83 (1H, d, *J* = 10.4 Hz), 4.02 (6H, s), 4.01 (3H, s), 3.43-3.51 (2H, m), 3.12-3.21 (2H, m), 2.83 (3H, s), 2.75 (6H, s).

### Example 67

### N-(5-(4-(2,6-dichloro-3,5-dimethoxyphenyl)imidazo[1,2-a][1,6]naphthyridin-8-yl)-2-(4-dimethylamino)piperidin-1-yl)-4-methoxyphenyl)acrylamide

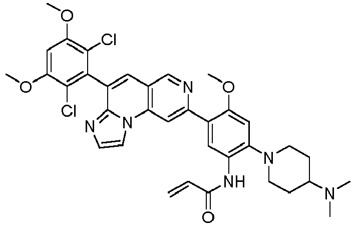

The synthetic method of example 67 was same as that of example 54. ¹H NMR (400 MHz, CDCl₃) δ 9.18 (1H, s), 8.92 (1H, s), 8.37 (1H, s), 8.12-8.18 (2H, m), 7.69 (1H, s), 7.49 (1H, s), 6.79 (1H, s), 6.71 (1H, s), 6.42-6.45 (2H, m), 5.77-5.80 (1H, m), 3.98 (6H, s), 3.90 (3H, s), 3.31 (2H, d, *J* = 12.0 Hz), 3.12-3.24 (1H, m), 2.79-2.89 (8H, m), 2.35 (2H, d, *J* = 11.2 Hz), 2.07-2.22 (2H, m).

### Example 68

### N-(5-(4-(2,6-dichloro-3,5-dimethoxyphenyl)imidazo[1,2-a][1,6]naphthyridin-8-yl)-4-methoxy-2-(8-oxa-2-azaspiro[4.5]decan-2-yl)phenyl)acrylamide

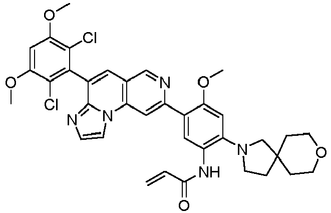

The synthetic method of example 68 was same as that of example 54. ¹H NMR (400 MHz, CDCl₃) δ 9.13 (1H, s), 8.40 (1H, s), 8.29 (1H, s), 8.12 (1H, s), 7.78 (1H, s), 7.68 (1H, s), 7.47 (1H, s), 6.70 (1H, s), 6.44 (1H, s), 6.42 (1H, d, *J* = 16.8 Hz), 6.34 (1H, dd, *J* = 16.8, 10.0 Hz), 5.77 (1H, d, *J* = 10.0 Hz), 3.97 (6H, s), 3.94 (3H, s), 3.61-3.71 (4H, m), 3.36 (2H, t, *J* = 6.8 Hz), 3.14 (2H, s), 1.81 (2H, t, *J* = 6.8 Hz), 1.52-1.68 (4H, m).

### Example 69

### N-(5-(4-(2,6-dichloro-3,5-dimethoxyphenyl)imidazo[1,2-a][1,6]naphthyridin-8-yl)-2-(4-ethylpiperazin-1-yl)-4-methoxyphenyl)acrylamide

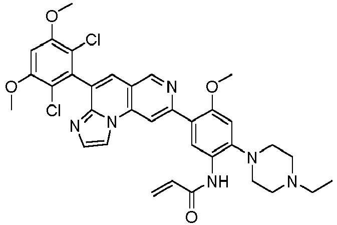

The synthetic method of example 69 was same as that of example 54. ¹H NMR (400 MHz, CDCl₃) δ 9.21 (1H, s), 9.04 (1H, s), 8.37 (1H, s), 8.32 (1H, s), 8.12 (1H, s), 7.69 (1H, s), 7.50 (1H, s), 6.91 (1H, s), 6.71 (1H, s), 6.42 (1H, d, *J* = 16.8 Hz), 6.30 (1H, dd, *J* = 16.8, 10.4 Hz), 5.77 (1H, d, *J* = 10.4 Hz), 3.98 (6H, s), 3.90 (3H, s), 3.01-3.05 (4H, m), 2.58-2.75 (4H, m), 2.54 (2H, q, *J* = 7.2 Hz), 1.17 (3H, t, *J* = 7.2 Hz).

### Reference Example 70

### N-(5-(6-(2,6-dichloro-3,5-dimethoxyphenyl)imidazo[1',2':1,6]pyrido[2,3-d]pyrimidin-2-yl)-4-methoxy-2-(4-(oxetan-3-yl)piperazin-1-yl)phenyl)acrylamide

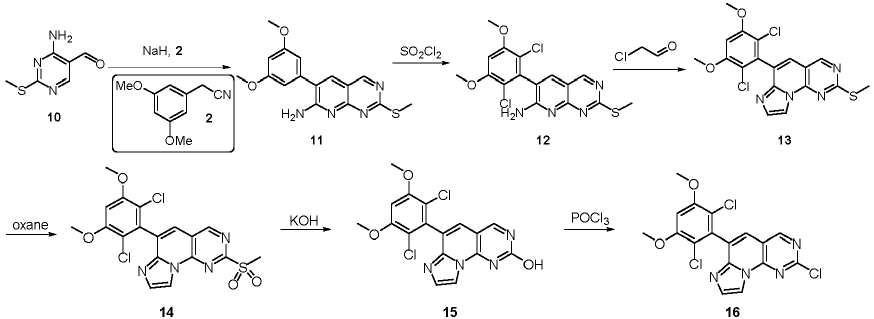

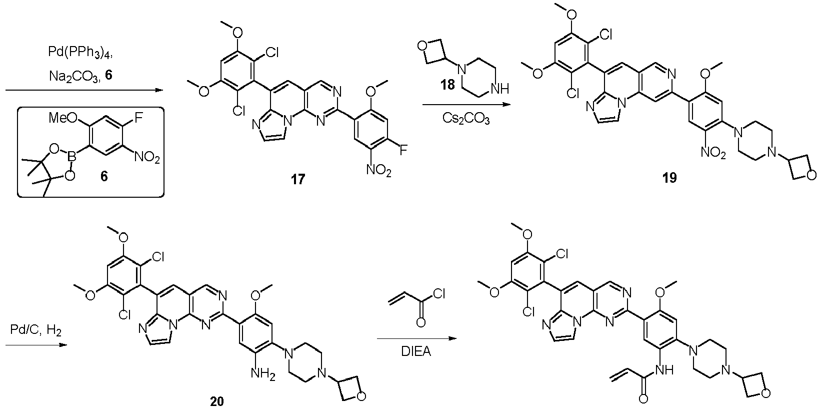

### Step 1: synthesis of compound 11

At room temperature, to a solution of compound **2** (3.0 g) in tetrahydrofuran (20 mL) was added sodium hydride (774 mg), the mixture was stirred at room temperature for 1.5 hours, then compound **10** (2.86 g) was added into the mixture, and the mixture was stirred at room temperature overnight. An aqueous solution of saturated ammonium chloride was used to quench the reaction, and dichloromethane was used for dilution, the organic phase was separated, washed with aqueous solution of saturated ammonium chloride, dried over anhydrous sodium sulfate, filtered, and concentrated, and the residue was purified through flash silica gel column chromatography (DCM/MeOH = 100/1) to obtain compound **11** (5.2 g).

### Step 2: synthesis of compound 12

At -20 °C, to a solution of compound **11** (5.2 g) in tetrahydrofuran (100 mL) was slowly dropwise added sulfuryl chloride (2.81 g). After the addition was complete, the mixture was stirred at -20 °C for 2 hours. Then an aqueous solution of saturated sodium bicarbonate was used to quench the reaction. The mixture was extracted with ethyl acetate, the extract was dried over anhydrous sodium sulfate, filtered and concentrated, and the residue was purified through flash silica gel column chromatography (DCM/MeOH = 100/2) to obtain compound **12** (4.7 g).

### Step 3: synthesis of compound 13

To a solution of compound **12** (2.0 g) in ethanol (20 mL) was added an aqueous solution of 40% chloroacetaldehyde (8 mL), and the mixture was heated at 80 °C overnight. The mixture was poured into an aqueous solution of saturated sodium bicarbonate, stirred and filtered, and the filter cake was dried to obtain a light yellow solid **13** (2.1 g). This compound was directly used in the next step without further purification.

### Step 4: synthesis of compound 14

To a solution of compound **13** (2.1 g) in dichloromethane/methanol/water (20 mL/ 20 mL/ 20 mL) was added potassium peroxymonosulfate sulfate (18.4 g), and the mixture was heated to 40 °C and stirred overnight. Water was added to dilute the mixture, the mixture was extracted with dichloromethane, and the extract was dried over anhydrous sodium sulfate, filtered and concentrated. The light yellow solid **14** thus obtained was used in the next step.without further purification.

### Step 5: synthesis of compound 15

To a solution of compound **14** (2.5 g) in THF (10 mL) was added a solution of potassium hydroxide (924 mg) in water (10 mL). The reaction solution was stirred at room temperature overnight. The mixture was concentrated under reduced pressure to remove THF, and then concentrated hydrochloric acid was added to pH = 2. The mixture was filtered, the solid thus obtained was washed with anhydrous acetonitrile (5 mL) and dried to obtain compound **15** (2.1 g).

### Step 6: synthesis of compound 16

To a solution of compound **15** (2.1 g) in acetonitrile (20 mL) was added phosphorus oxychloride (20 mL). The reaction solution was heated to 80 °C and reacted for 3 hours, then cooled to room temperature. The reaction solution was concentrated under reduced pressure, then ice water was added. The mixture was filtered, and thus obtained solid was washed with anhydrous acetonitrile and dried to obtain compound **16** (1.9 g).

### Step 7: synthesis of compound 17

Under nitrogen protection, to a solution of compound **16** (1.14 g) in dioxane/water (20 mL/5 mL) were added tetrakis(triphenylphosphine)palladium (500 mg), compound **6** (1.3 g) and anhydrous sodium carbonate (1.2 g). The reaction solution was heated to 80 °C and reacted overnight. The reaction solution was cooled to room temperature and concentrated under reduced pressure, and the residue thus obtained was separated and purified through flash silica gel column chromatography (dichloromethane/methanol = 500/1) to obtain compound **17** (920 mg).

### Step 8: synthesis of compound 19

To a solution of compound **17** (200 mg) in DMF (1 mL) were added anhydrous caesium carbonate (120 mg) and compound **18** (120 mg), and the mixture was heated to 60 °C and reacted overnight. The reaction solution was cooled to room temperature and concentrated under reduced pressure. The residue thus obtained was separated and purified through flash silica gel column chromatography (dichloromethane/methanol = 100/1 to 50/1) to obtain compound **19** (110 mg).

### Step 9: synthesis of compound 20

To a solution of compound **19** (110 mg) in tetrahydrofuran (5 mL) was added palladium on carbon (50 mg), and the system was purged with hydrogen gas. The reaction system was reacted at room temperature overnight and filtered through celite. The filtrate was concentrated under reduced pressure to obtain compound **20** (63 mg).

### Step 10: synthesis of example 70

In an ice bath, to a solution of compound **20** (63 mg) in dichloromethane (2 mL) were added DIEA (5 µL) and acryloyl chloride (11 µL). The reaction solution was stirred in an ice bath for 2 hours, then methanol was added to quench the reaction, and the mixture was concentrated under reduced pressure. The residue thus obtained was separated and purified through preparative thin layer chromatography (dichloromethane/methanol = 20/1) to obtain example 70 (33 mg). ¹H NMR (400 MHz, CDCl₃) δ 9.34 (1H, s), 9.07 (1H, s), 8.58 (1H, s), 8.26 (1H, s), 7.68 (1H, s), 7.43 (1H, s), 6.93 (1H, s), 6.71 (1H, s), 6.42 (1H, d, *J* = 16.8 Hz), 6.28 (1H, dd, *J* = 16.8, 10.4 Hz), 5.78 (1H, d, *J* = 10.4 Hz), 4.74 (2H, t, *J* = 6.4 Hz), 4.68 (2H, t, *J* = 6.4 Hz), 3.98 (6H, s), 3.93 (3H, s), 3.61-3.67 (1H, m), 3.03-3.09 (4H, m), 2.51-2.65 (4H, m).

### Bioactivity assay:

### 1. In vitro determination of the protein activity of compounds:

The FGFR-4 kinase activity detection platform was established by homogeneous time-resolved fluorescence (HTRF) method, and the activity of compound was determined. 1000 µM compounds were subjected to 3-fold gradient dilution in 100% DMSO 11 times (12 concentrations in total), 4 µL of diluent from each concentration was added to 96 µL of reaction buffer (50 mM HEPES, pH 7.4, 5 mM MnCl₂, 0.1 mM NaVO₃, 0.001% Tween-20, 0.01% BAS, 1 mM DTT) and mixed well, as a 4* compound (final concentration of 0.017 nM-1000 nM) for later use. 2 * FGFR-4 kinase (final concentration of 1 nM) was formulated using reaction buffer and 4 * substrate ( ATP+TK peptide)( TK peptide, HTRF^{®} KinEASE^{™}-TK, purchased from Cisbio, TK peptide, final concentration of 1 µ M, ATP final concentration of 25 µ M) was formulated using reaction buffer. 2.5 µL of 4 * compound and then 5 µL of 2 * FGFR-4 kinase were added to a 384-well plate ( OptiPlate-384, purchased from PerkinElmer), the mixture was mixed by centrifugation, and 2.5 µL of 4 * substrate mixture was added to start the reaction (total reaction volume of 10 µL). The 384-well plate was placed in an incubator 23°C and stirred for 60 min before the reaction was stopped by the addition of 5 µL of Eu³⁺ cryptate-labled anti-phosphotyrosine antibody (TRF^{®} KinEASE^{™}-TK, purchased from Cisbio) and 5 µL of Streptavidin-XL-665 (HTRF^{®} KinEASE^{™}-TK, purchased from Cisbio). After incubation in the incubator for 1 h, fluorescence values (excitation at 320 nm, detecting emitted light at 665 nm and 620 nm, and a ratio of the two as enzyme activity) were read on Envision (purchased from PerkinElmer). The enzyme activity of each compound was measured at 12 concentrations, and the IC₅₀ value of the compound was calculated using GraphPad prism 5.0 software.

### 2. Determination of cell proliferation activity of compounds:

Cell Titer-Glo ^{®} detection reagent from Promega was used to establish the suspension cell proliferation inhibition screening method. Human hepatoma cells Hep3b (Xiehe Cell Research Center) were cultured in 25 cm² or 75 cm² plastic tissue culture flasks (Corning^{®}) filled with MEM (Gibco^{®}) medium supplemented with 10% fetal bovine serum (Hyclone^{®}) under 37°C, 95% air and 5% CO₂, and subcultured 2-3 times a week.

Hep3b cells were seeded in a 96-well cell culture plate (Coming^{®}) at a density of 3×10³ cells/well, 195 mL/well, and cultured under 37°C, 95% air, and 5% CO₂. After 24 h the test compound was added: 10 mM compounds (dissolved in DMSO) were subjected to 3-fold gradient dilution in DMSO, 4 mL of diluent from each concentration was added to 96 mL of serum-free medium, and finally, 5 mL of compound diluted in the medium was added to the plate seeded with cells. The final concentration of DMSO in the cell culture medium was 0.1% and the final concentration of test compound was 0.3 nM-10 mM. The above cells were incubated for 3 days under 37°C.

After 3 days, the cell viability assay was performed by the Cell Titer-Glo (Promega) kit, and finally the inhibitory concentration 50% of the compound on cell proliferation, i.e. the IC 50 value, was calculated by the GraphPad Prism 5.0 program.

**Table 2. Inhibitory effects of the compounds in Examples on FGFR4 and Hep3B cell lines**

| Compound | FGFR4 IC₅₀ (nM) | Hep3B IC₅₀ (nM) |
|---|---|---|
| Example 54 | 0.7 | 21.5 |
| Example 59 | 1.1 | 63.2 |
| Example 60 | 0.9 | 95.2 |
| Example 61 | 1.7 | 121 |
| Example 62 | 2.4 | 43.8 |
| Example 64 | 0.3 | 69.7 |
| Example 65 | 0.9 | 22.3 |
| Example 67 | 0.4 | 81.3 |
| Blu-554 | 2.21 | 66.1 |

### 3. Pharmacokinetic data

Male SD rats (Beijing Vital River Laboratory Animal Technology Co., Ltd.) were divided into groups of 3 rats each. The rats were subjected to 5 mg/kg suspension of the test sample by single intragastric administration, respectively. Animals were fasted overnight prior to the experiment from 10 h before dosing to 4 h after dosing. Blood was collected 0.25, 0.5, 1, 2, 4, 6, 8, and 24 h after administration. After isoflurane anesthesia using a small animal anesthesia machine, 0.3 mL of whole blood samples was taken through the fundus venous plexus, placed in a heparin anticoagulant tube, and were centrifuged at 4°C, 4000 rpm for 5 min, plasma was transferred to the centrifuge tube, and stored at -80°C until analysis. Samples in the plasma were extracted using protein precipitation and the extracts were analyzed by LC/MS/MS.

| Example | Example 59 | Example 62 | Example 61 | Blu-554 |
|---|---|---|---|---|
| Dose (mg/kg) | 5 | 5 | 5 | 5 |
| T1/2 (hr) | 3.97 | 4.93 | 2.60 | 1.64 |
| Tmax (hr) | 2.67 | 5.33 | 1.17 | 2.67 |
| Cmax (ng/mL) | 505 | 175 | 561 | 362 |
| AUC0-inf (hr*ng/mL) | 3942 | 1118 | 3048 | 1683 |

### Industrial Applicability

The present invention provides an FGFR4 kinase inhibitor, a preparation method therefor and use thereof, as further defined in the claims. The present invention relates to the compounds as defined in the claims or a pharmaceutically acceptable salt, solvate, polymorph, or isomer thereof, and use thereof in the preparation of a drug for the treatment of FGFR4-mediated diseases. The FGFR4 protein kinase inhibitor can inhibit FGFR4 tyrosine kinase with high selectivity, has a weak inhibition effect on FGFR1-3, to safely and effectively treat liver cancer patients with high FGFR4 expression, and has good economic value and application prospect.

## Claims

1. The compound below, or a pharmaceutically acceptable salt, solvate, polymorph or tautomer thereof:

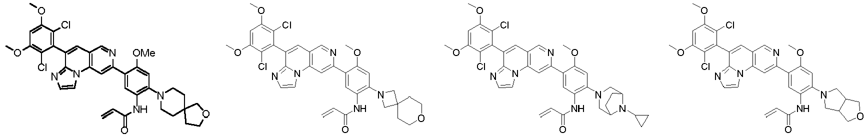

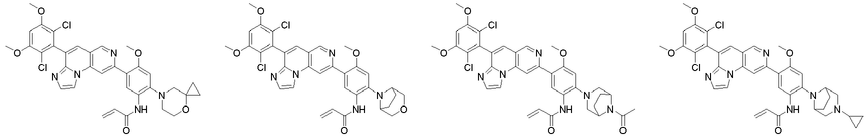

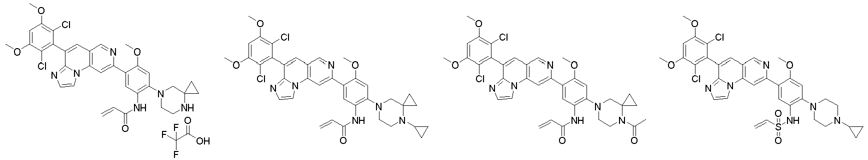

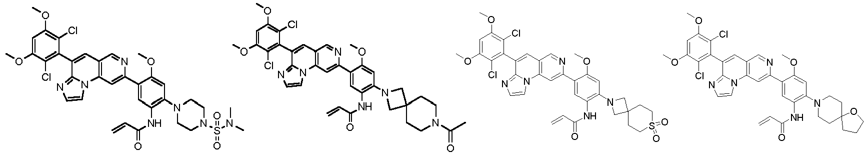

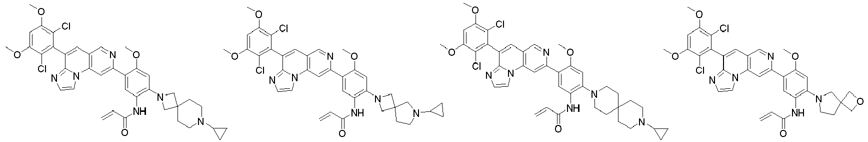

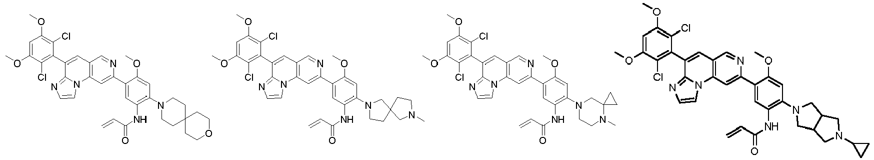

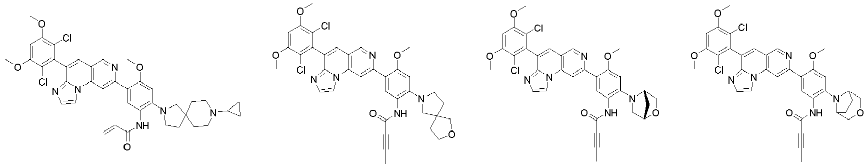

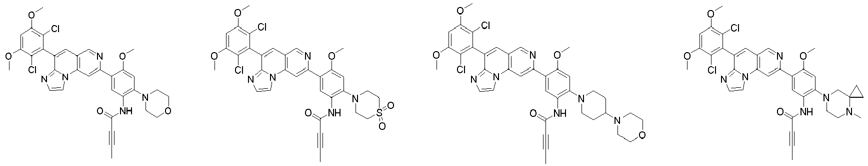

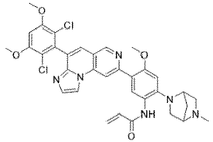

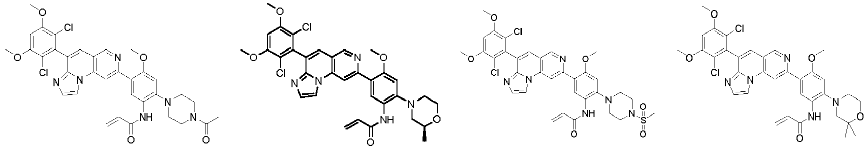

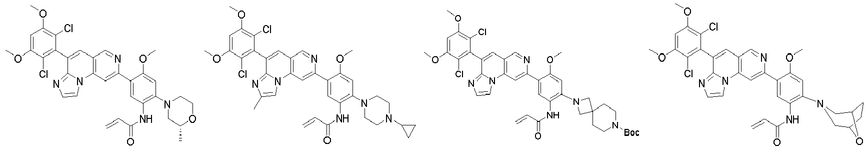

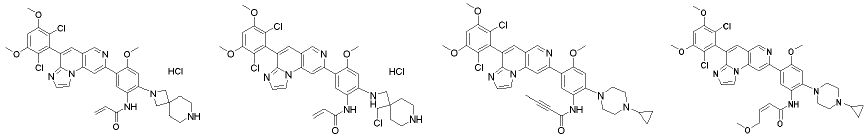

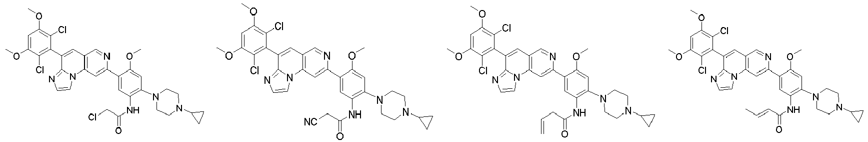

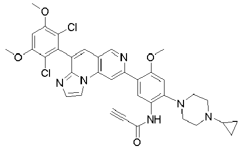

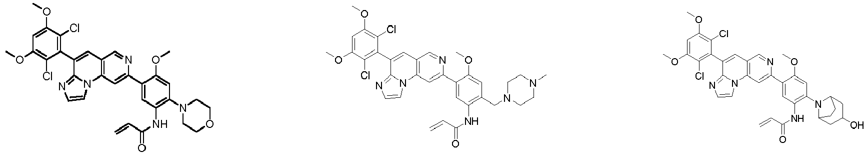

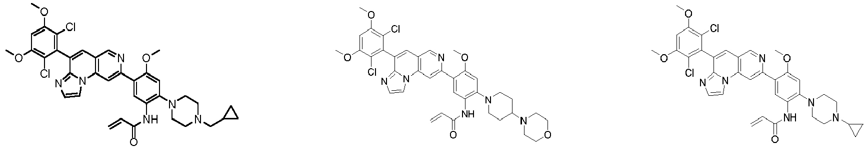

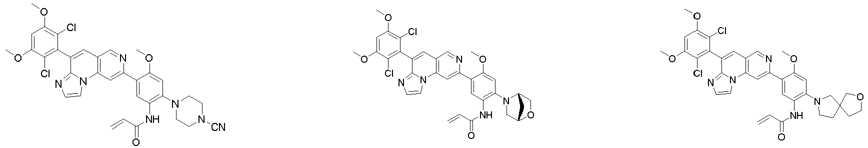

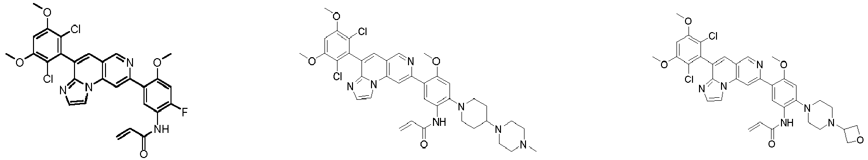

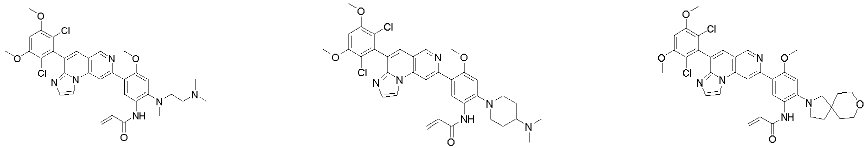

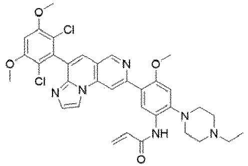

2. A composition comprising the compound according to claim 1 or a pharmaceutically acceptable salt, solvate, polymorph or tautomer thereof, and a pharmaceutically acceptable carrier.

3. The compound according to claim 1 or a pharmaceutically acceptable salt, solvate, polymorph or tautomer thereof and the composition according to claim 2 for use in the treatment of an FGFR4 mediated disease, wherein the FGFR4 mediated disease is nonsmall cell lung cancer, gastric carcinoma, multiple myeloma, liver cancer, cholangiocarcinoma, prostate cancer, skin cancer, ovarian cancer, breast cancer, colon cancer, glioma, and rhabdomyosarcoma.

## Patentansprüche

1. Nachstehende Verbindung oder ein pharmazeutisch akzeptables Salz, Solvat, Polymorph oder Tautomer davon:

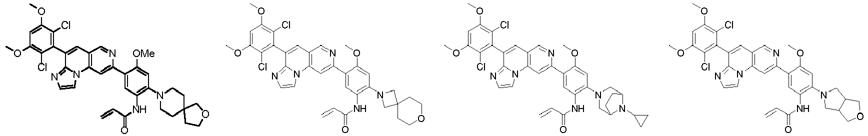

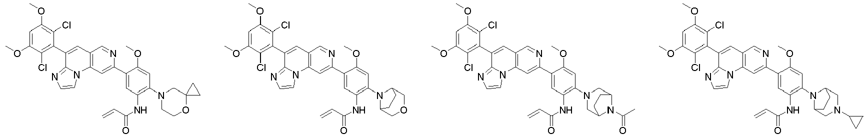

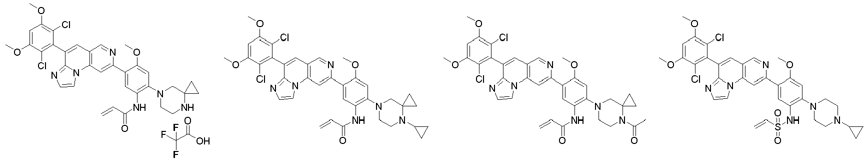

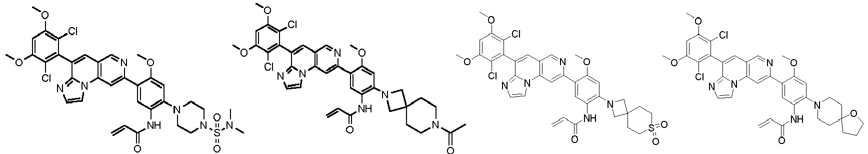

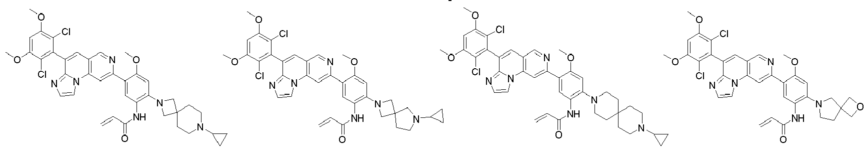

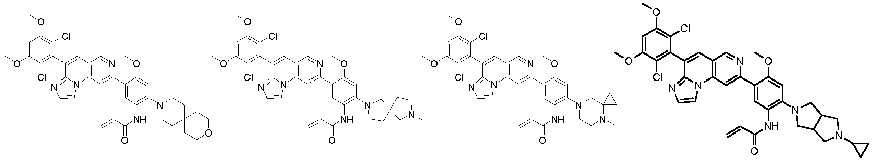

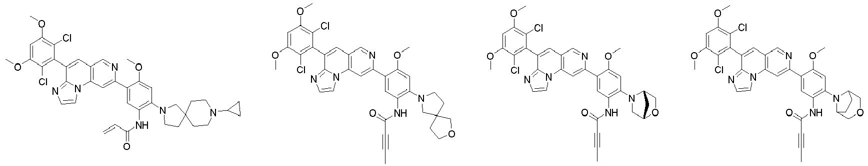

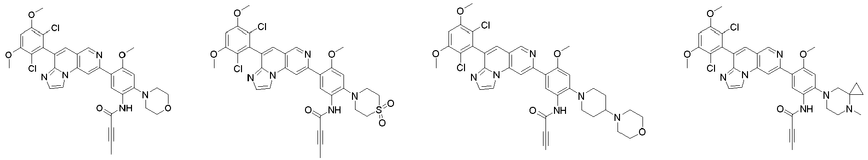

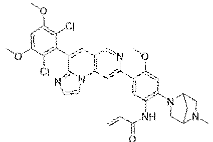

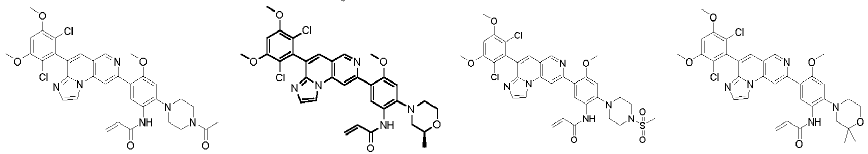

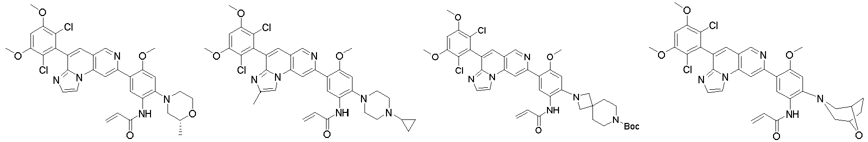

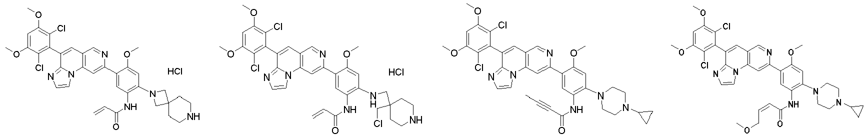

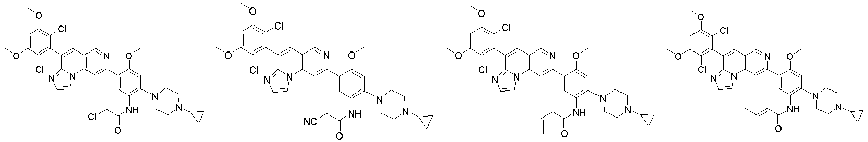

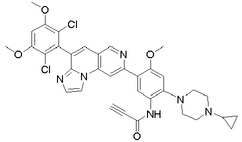

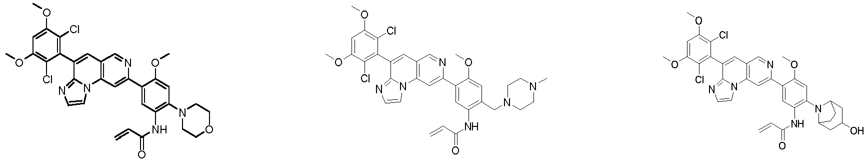

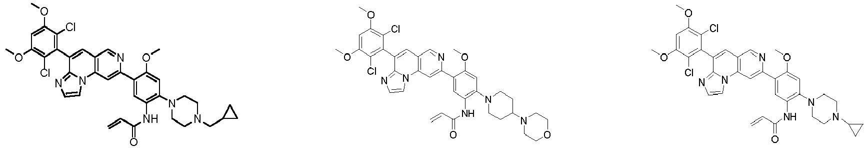

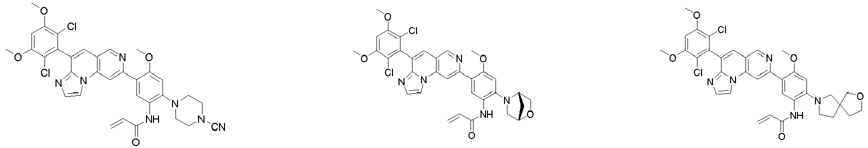

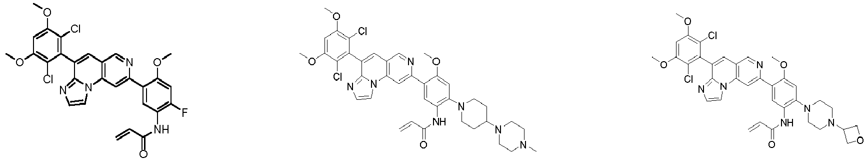

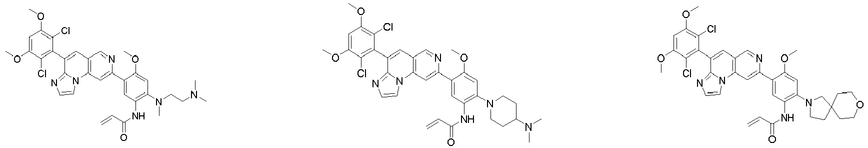

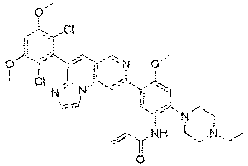

2. Zusammensetzung, umfassend die Verbindung gemäß Anspruch 1 oder ein pharmazeutisch akzeptables Salz, Solvat, Polymorph oder Tautomer davon und einen pharmazeutisch akzeptablen Träger.

3. Verbindung gemäß Anspruch 1 oder ein pharmazeutisch akzeptables Salz, Solvat, Polymorph oder Tautomer davon und die Zusammensetzung gemäß Anspruch 2 zur Verwendung bei der Behandlung einer FGFR4-bedingten Krankheit, wobei die FGFR4-bedingte Krankheit nicht-kleinzelliger Lungenkrebs, Magenkarzinom, multiples Myelom, Leberkrebs, Cholangiokarzinom, Prostatakrebs, Hautkrebs, Eierstockkrebs, Brustkrebs, Dickdarmkrebs, Gliom und Rhabdomyosarkom ist.

## Revendications

1. Composé ci-dessous, ou sel, solvate, polymorphe ou tautomère pharmaceutiquement acceptable de celui-ci :

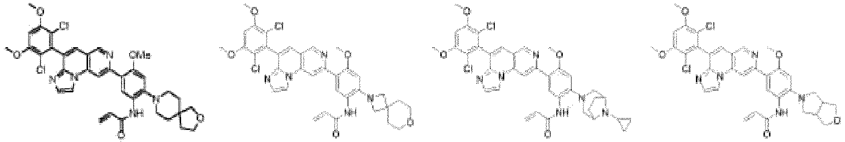

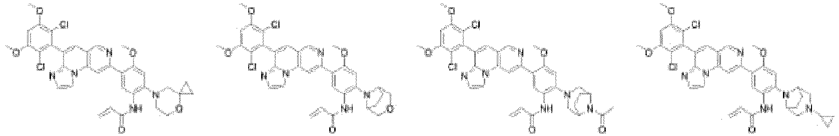

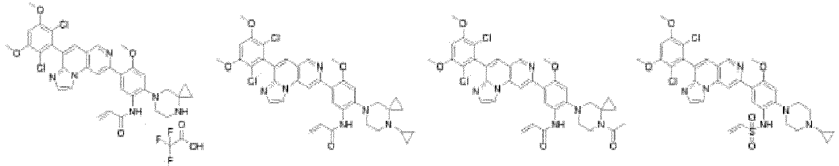

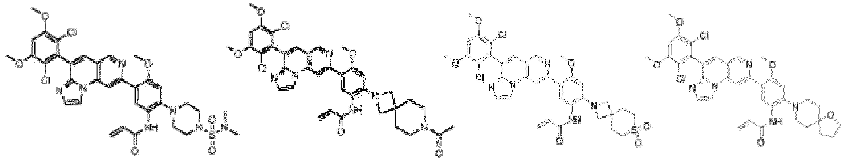

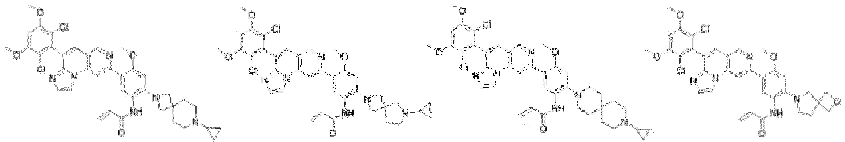

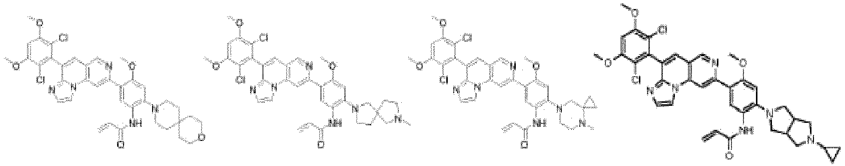

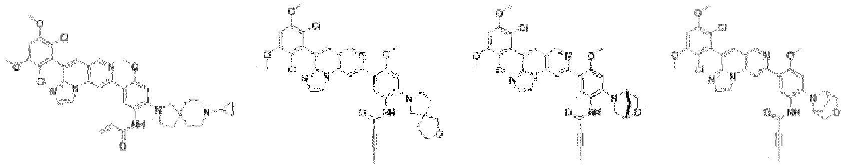

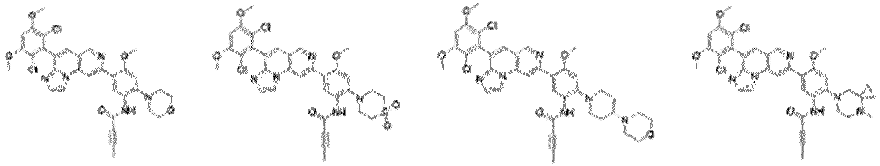

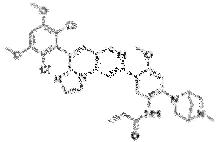

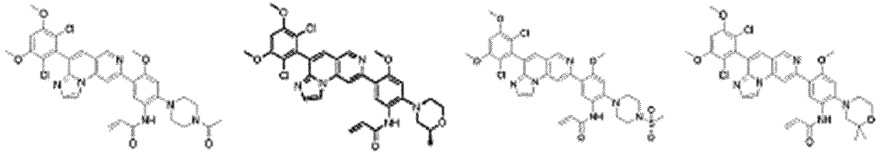

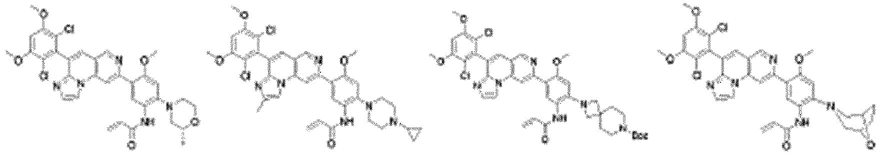

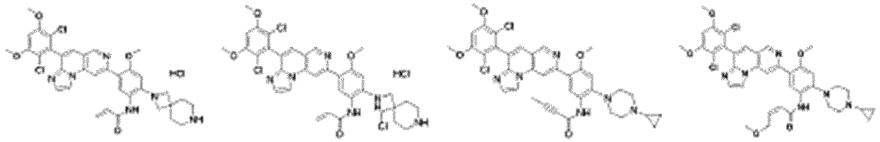

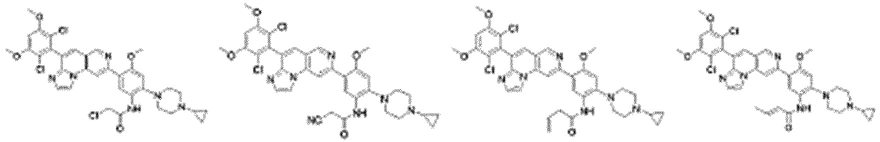

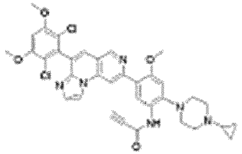

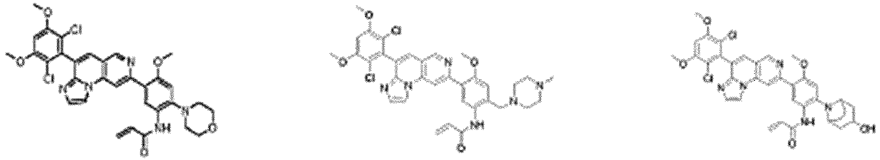

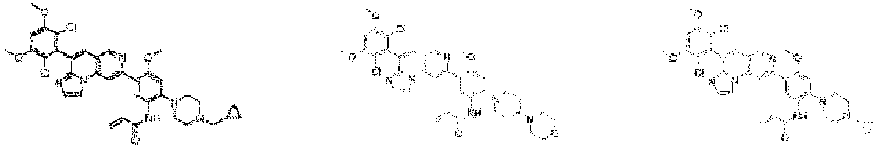

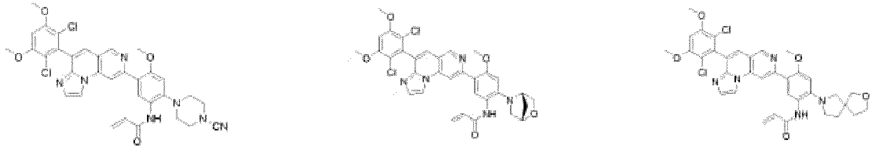

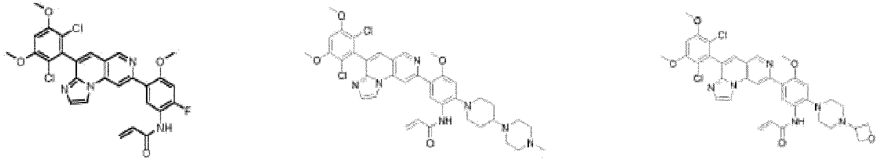

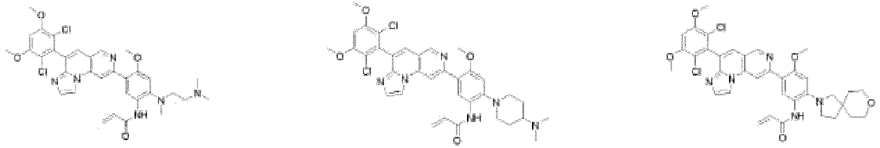

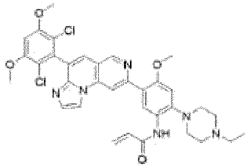

2. Composition comprenant le composé selon la revendication 1 ou un sel, un solvate, un polymorphe ou un tautomère pharmaceutiquement acceptable de celui-ci, et un support pharmaceutiquement acceptable.

3. Composé selon la revendication 1 ou sel, solvate, polymorphe ou tautomère pharmaceutiquement acceptable de celui-ci et composition selon la revendication 2 pour utilisation dans le traitement d'une maladie médiée par le FGFR4, dans lequel la maladie médiée par le FGFR4 est le cancer bronchique non à petites cellules, le carcinome gastrique, le myélome multiple, le cancer du foie, le cholangiocarcinome, le cancer de la prostate, le cancer de la peau, le cancer de l'ovaire, le cancer du sein, le cancer du côlon, le gliome et le rhabdomyosarcome.
